# EUROPEAN PATENT APPLICATION

(11) **EP 4 427 740 A1**
(43) Date of publication of application: **11.09.2024**
(21) Application number: 22890484.3
(22) Date of filing: 07.11.2022
(51) Int. Cl.: A61K 9/16, A61K 9/00, A61K 45/06, A61P 29/00, A61K 31/445, A61K 31/573

(54) **PHARMACEUTICAL KIT FOR PARENTERAL CO-ADMINISTRATION**

(30) Priority: 05.11.2021 KR 20210151652; 01.09.2022 KR 20220110883
(71) Applicant: G2GBIO, Inc., Cheongju-si, Chungcheongbuk-do 28161 (KR)
(72) Inventor: KIM, Geonho, Cheongju-si, Chungcheongbuk-do 28222 (KR); LEE, Jinwoo, Sejong-si, 30144 (KR); JUNG, Hyejung, Seoul 03780 (KR); CHOI, Jaemook, Sejong-si, 30101 (KR); BYUN, Jeongsu, Daejeon 34127 (KR); LEE, Juhan, Daejeon 35218 (KR); SEOL, Eunyoung, Daejeon 34080 (KR); LEE, Heeyong, Daejeon 34032 (KR)
(74) Representative: Ricker, Mathias
(86) International application number: PCT/KR2022/017384
(87) International publication number: WO 2023/080753

(57) **Abstract**

The present invention is a pharmaceutical kit for combined parenteral administration, comprising a first formulation containing a first drug, and a second formulation containing a second drug and a parenteral drug delivery system, and it can be used for preventing, reducing or treating an inflammatory reaction of a subject caused by the parenteral drug delivery system of the second formulation, controlling decomposition of the parenteral drug delivery system contained in the second formulation, or increasing bioavailability of the second drug contained in the second formulation.

## Description

### [Technical field]

The present invention relates to a pharmaceutical kit for combined parenteral administration, which improves the bioavailability of concomitantly administered drugs using an anti-inflammatory drug, or prevents, alleviates or treats an inflammatory reaction caused by a combined administered parenteral drug delivery system, or to control the decomposition of a parenteral drug delivery system administered in combination.

### [Related Art]

For various reasons, such as increased drug efficacy, reduced side effects, or administration convenience, a number of drugs administered in combination are being developed in which two or more drugs are administered in various ways.

In addition, a drug delivery system (DDS) is being used for the purpose of maximizing the therapeutic effect of drugs and minimizing side effects on the human body. Drug delivery systems include drugs and simple formulations to highly functional formulations, and are applied to patients through various routes in the human body, such as oral, injection, transdermal, mucosal, and transplantation. In particular, development of various types of drug preparations for parenteral administration, which are loaded with drugs, is actively progressing in order to improve the patient's ease of taking and improve the therapeutic effect of drugs that must be taken for a long period of time for the treatment of diseases.

However, in the case of these formulations, a technology is applied in which a substance that does not exist in the body is used as a drug delivery system, and a long-term therapeutic agent is released from the body by loading the target drug. It may induce an undesirable reaction, such as an immune response or an inflammatory response.

This inflammatory response is maintained continuously until the parenteral drug delivery system loaded with the target drug is completely decomposed in the body, and thus, it is highly likely that acute inflammation will progress to chronic inflammation. Accordingly, there is a need for an anti-inflammatory formulation or kit for preventing, reducing or treating the occurrence of inflammation in a subject administered by a drug delivery system.

On the other hand, if excessive drug release or release at an inappropriate time occurs in a long-acting drug formulation, the target drug release profile cannot be achieved, resulting in side effects or the inability to secure the expected drug efficacy.

In addition, when a drug is to be continuously released for a long period of time, a large amount of drug that can exhibit sufficient drug efficacy for a target period must be introduced into the drug delivery system. If the bioavailability of the drug contained in the formulation is low, administration of an excessively large amount of the drug delivery system is required to exhibit effective pharmacological effects over a long period of time. However, when a large amount of drug carrier is administered in vivo, it is difficult for the patient to administer directly (self-administration) because it cannot be applied by subcutaneous injection, or it may be necessary to divide and inject in several areas, causing pain and inflammatory reactions at the injection site. It can cause escalating problems.

### [Detailed Description]

### [Problem to be solved]

The present invention relates to a pharmaceutical kit for combined parenteral administration, which improves bioavailability of co-administered drugs; prevents, alleviates or treats an inflammatory reaction caused by a co-administered parenteral drug delivery system; or controls decomposition of a co-administered parenteral drug delivery system, by using an anti-inflammatory drug.

An embodiment of the present invention provides a pharmaceutical kit for combined parenteral administration or its use for preventing, alleviates or treating an inflammatory reaction caused by a parenteral drug delivery system administered in combination with an anti-inflammatory drug, by using an anti-inflammatory drug.

A further embodiment of the present invention provides a pharmaceutical kit for combined parenteral administration or its use for controlling decomposition rate of co-administered parenteral drug delivery system, by using an anti-inflammatory drug.

Another embodiment of the present invention provides a pharmaceutical kit for combined parenteral administration or its use for increasing bioavailability of a co-administered drug, by using an anti-inflammatory drug.

Another embodiment of the present invention relates to a parenteral pharmaceutical composition or kit, further including a local anesthetic component in addition to the pharmaceutical kit for combined parenteral administration.

### [Means to solve problems]

An embodiment of the present invention is a pharmaceutical kit for combined parenteral administration or use thereof, comprising a first formulation containing a first drug and a second formulation including a second drug and a parenteral drug delivery system,
the first formulation includes a first anti-inflammatory drug, or a first anti-inflammatory drug loaded in a first parenteral drug delivery system, and the second formulation includes a second parenteral drug delivery system and a second drug being different from the first drug, wherein the first formulation is for preventing, alleviating or treating an inflammatory reaction of a subject which is caused by the parenteral drug delivery system of the second formulation.

An embodiment of the present invention is a pharmaceutical kit for combined parenteral administration or use thereof, comprising a first formulation containing a first drug and a second formulation including a second drug and a parenteral drug delivery system,
the first formulation includes a first anti-inflammatory drug, or a first anti-inflammatory drug loaded in a first parenteral drug delivery system, and the second formulation includes a second parenteral drug delivery system and a second drug being different from the first drug, wherein the first formulation is for controlling decomposition of the parenteral drug delivery system included in the second formulation.

An embodiment of the present invention is a pharmaceutical kit for combined parenteral administration or use thereof, comprising a first formulation containing a first drug and a second formulation including a second drug and a parenteral drug delivery system,
the first formulation includes a first anti-inflammatory drug, or a first anti-inflammatory drug loaded in a first parenteral drug delivery system, and the second formulation includes a second parenteral drug delivery system and a second drug being different from the first drug, wherein the first formulation is for increasing bioavailability of the second drug included in the second formulation.

The first formulation and the second formulation included in the pharmaceutical kit for combined parenteral administration may be administered at the same time or at different times. The pharmaceutical kit for combined parenteral administration may include the first formulation and the second formulation in the form of a mixed formulation or separate formulation.

The pharmaceutical kit for combined parenteral administration according to the present invention may include a first formulation containing a first drug, and a second formulation containing a second drug and a parenteral drug delivery system.

The first drug contained in the first formulation may be an anti-inflammatory drug, and the first drug is different from the second drug contained in the second formulation. In addition, the first drug may be provided without an individual drug delivery system, provided together with the drug delivery system of the second formulation, or separately supported in the first parenteral drug delivery system.

For example, when the first drug contained in the pharmaceutical kit is provided without an individual drug delivery system, the first drug may be provided in the form of a mixed formulation of the second formulation including a second drug and a second parenteral drug delivery system together with the first drug, or only the first drug may be provided separately from the second formulation at different times. Specifically, when the second formulation contains microparticles, the first drug and the second drug may be a formulation supported together in one microparticle.

Alternatively, the first drug contained in the pharmaceutical kit may be provided in a form being supported in an individual first drug delivery system, and the first drug delivery system may be the same as or different from the second drug delivery system. Specifically, when the first formulation contains the first drug loaded in the first parenteral drug delivery system, it may be provided in the form of a mixed formulation or a separate formulation with the second formulation, and can be administered at the same time or at different times to a subject in need thereof.

In another embodiment of the present invention, AUC of the anti-inflammatory drug at the blood concentration being equal to or higher than the lowest effective blood concentration of the anti-inflammatory drug released from the first anti-inflammatory formulation is 1/2 or less, 1/3 or less, 1/5 or less or 1/10 or less of the total AUC of the daily effective dose of the drug, or 50% or less, 30% or less, 20% or less, or 10% or less, based on 100% of the total AUC of the daily effective dose of the drug. For example, when the anti-inflammatory first formulation containing dexamethasone is administered to human body, AUC at the blood dexamethasone concentration being equal to or higher than 1 ng/mL, is 1/2 or less, preferably 1/3 or less, more preferably 1/5 or less, and most preferably 1/10 or less than the total AUC of the daily oral dexamethasone dose of 6 mg.

In a specific example, the anti-inflammatory drug released from the first anti-inflammatory formulation may be released so that the plasma concentration of the anti-inflammatory drug is 1.0 ng/mL or less, in which case a preferred example of the anti-inflammatory drug may be dexamethasone or a derivative thereof, or pharmaceutical salts thereof.

According to an embodiment of the present invention, the first drug (anti-inflammatory drug) included in the first formulation may often cause side effects in the sustained-release formulation, when the blood concentration of drug is excessively high. For example, when the anti-inflammatory drug is a glucocorticoid, high plasma concentrations (1 mg/kg/day for 2-3 weeks) can cause systemic side effects often. Thus, it is preferable to achieve low plasma concentration compared to the concentration at the local administration site.

In another embodiment of the present invention, in the blood concentration curve over time after administration of the anti-inflammatory drug released from the first anti-inflammatory formulation, AUC at a concentration being equal to or higher than the lowest blood concentration that causes systemic side effects, should be minimized, or specifically 1/10 or less, 1/20 or less, 1/30 or less, or 1/40 or less of the AUC obtained by single administering the same dose of the drug, or 10% or less, 5% or less, 3.3% or less, or 2.5% or less based on 100% of AUC obtained by single administering the same dose of the drug.

For example, when an anti-inflammatory first formulation containing dexamethasone or dexamethasone acetate is subcutaneously administered to rats, AUC in a concentration being equal to or higher than 2ng/ml or higher of the blood drug concentration is 1/10 or less, preferably 1/20 or less, more preferably 1/30 or less, and most preferably 1/40 or less, compared to AUC obtained by subcutaneously administering dexamethasone or dexamethasone acetate at the same dose.

In a specific example, the anti-inflammatory drug released from the first anti-inflammatory formulation may be released so that the plasma concentration of the anti-inflammatory drug is 2.0 ng/mL or less, in which a preferred example of the anti-inflammatory drug may be dexamethasone or a derivative thereof, or pharmaceutical salts thereof.

In the present specification, "drug delivery," "drug delivery system," "drug support" or "drug carrier" may be used interchangeably, and refers to a delivery system from which a pharmaceutically active ingredient can be released when being administered into body. The drug delivery systems applicable to the present invention are intended to include all parenteral drug delivery systems such as microspheres, liposomes, micelles, depots, or hydrogels. The hydrogel includes an injectable gel. The drug delivery system can be used as a carrier for delivering a drug to a living body, and can be made of biocompatible materials, preferably a biodegradable material for drug release. For example, materials of the drug delivery system include biodegradable polymers and biodegradable lipids, but are not limited to.

A drug delivery system applicable to the present invention may use a biocompatible material, and examples thereof include biodegradable polymers and biodegradable lipids, but are not limited to.

Specifically, the kind of biodegradable polymer is not particularly limited, but polyester may be preferably used. Examples of biodegradable polymers include at least one or preferable at least two selected from the group consisting of: at least one polymer selected from the group consisting of polylactide (PLA), polyglycolide (PGA), poly(lactide-co-glycolide, PLGA) as a copolymer of lactide and glycolide, polydioxanone, polycaprolactone (PCL), polylactide-co-glycolide-co-caprolactone (PLGC), Polylactide-co-hydroxymethyl glycolide (PLGMGA), Polyalkylcarbonate, Polytrimethylenecarbonate (PTMC), Polylactide-co-trimethylenecarbonate (PLTMC), Polyhydroxybutyric acid (PHB), Polyhydroxybutyrate-co-hydroxyvalerate (PHBV), Polyorthoester, Polyanhydride, Polyanhydride-co-imide, Polypropylene fumarate, Pseudo polyaminoacid, Polyalkyl cyanoacrylate, Polyphosphazene, Polyphosphoester, Polysaccharide and poly(butylene succinate lactide) (PBSLA); a simple mixture of two or more polymers selected therefrom; copolymer of polyethyleneglycol(PEG) and the polymer selected therefrom; and a polymer-saccharide complex which the polymer or the copolymer is linked to the saccharide. In a specific aspect, in the preparation method of the present invention, the biodegradable polymer can be poly(lactide-co-glycolide) and/or polylactide.

In the present specification, when the first formulation includes an anti-inflammatory drug and the first drug delivery system, the drug delivery system of the first formulation and the drug delivery system of the second formulation may be the same or different in material, size, dosage form, etc., and for example, may be the same dosage form. For example, the first drug delivery system and the second drug delivery system may be microspheres having various materials and/or sizes, respectively.

Herein, the drug delivery system of the first formulation is referred to a first parenteral drug delivery system, which is not particularly limited as long as it meets the desired release properties of the anti-inflammatory drug, and the first drug delivery system included in the anti-inflammatory formulation is preferably to have lower inflammation-inducing property and/or lower content.

The drug delivery system included in the second formulation is a drug delivery system used to deliver drugs used for prevention, improvement, or treatment of diseases, and may be a cause of inflammation. For example, it can be biodegradable polymers and biodegradable lipids, etc., but is not limited thereto.

Microspheres include microparticles, nanoparticles and the like, and are usually prepared by using biodegradable polymers. In an example, when the first drug delivery system is a microsphere, it may be a microsphere having an average particle diameter of 10 to 100 micrometers. The second drug delivery system may be microspheres, specifically microspheres having an average particle diameter of 10 to 100 micrometers.

An embodiment of the present invention relates to a parenteral anti-inflammatory kit for preventing, alleviating or treating an inflammatory reaction of a subject, where the inflammatory reaction is caused by the second drug delivery system of the second formulation,
which comprises a first anti-inflammatory formulation containing an anti-inflammatory drug and a first parenteral drug delivery system, and a second formulation containing a second parenteral drug delivery system, and
the first parenteral drug delivery system and the second parenteral drug delivery system include a biodegradable material, and the second parenteral drug delivery system is an inflammatory carrier that induces inflammation in a subject. The second formulation may further include a pharmaceutically active ingredient different from the anti-inflammatory drug of the first formulation.

In addition, the anti-inflammatory first formulation and the second formulation may be included as a mixed preparation or separate preparations, or may be administered at the same time or different times.

In the first formulation and the second formulation, the solid content of the first formulation may be 0.002 to 20 parts by weight, specifically 0.002 to 15 parts by weight, 0.002 to 10 parts by weight, and the like, based on 100 parts by weight of the solid content of the second formulation, wherein the second formulation may or may not contain a drug.

When the first formulation and the second formulation are provided as a mixed formulation, the anti-inflammatory drug may be included in an amount of 0.001 to 5.0 w/w%, or more specifically 0.001 to 5.0 w/w%, 0.001 to 4.0 w/w%, 0.001 to 3.0 w/w%, 0.001 to 2.0 w/w%, or 0.001 to 1.0 w/w%, based on 100% by weight of the solid content of the mixed formulation.

When the first formulation and the second formulation are provided as a mixed formulation, the first drug delivery system may be included in an amount of 0.002 to 20% by weight based on 100% by weight of the solid content of the mixed formulation.

When the pharmaceutical kit for combined parenteral administration according to an embodiment of the present invention is for preventing, alleviating or treating an inflammatory reaction in a subject which is caused by the parenteral drug delivery system of the second formulation, the kit may include the first anti-inflammatory formulation containing an anti-inflammatory drug as a first drug, or containing an anti-inflammatory drug and a first parenteral drug delivery system, and a second formulation containing a second drug being different from the anti-inflammatory drug of the first formulation where the second drug is supported in the second parenteral drug delivery system. In this case, the anti-inflammatory first drug contained in the first anti-inflammatory formulation may be released during the same period as the administration interval of the second formulation or during a part of the administration interval of the second formulation, so as to achieve anti-inflammatory efficacy.

As another embodiment, the anti-inflammatory drug contained in the first anti-inflammatory formulation is released at a relatively uniform concentration during the release period, or at a relatively high concentration at a certain time, for example, at an initial period or a later period of 50% or less, 40% or less, 30% or less, 20% or less, or 10% or less of the entire administration interval of the second formulation, depending on the properties required by the second formulation. In another example, the anti-inflammatory drug contained in the first anti-inflammatory formulation may be released at a concentration which is proportional to the remaining amount of the second parenteral drug delivery system contained in the second formulation. Specifically, the drug release amount of the first formulation gradually decreases depending on the relative content (% by weight) of the second drug delivery system remaining at the administration site, based on 100 % by weight of the content of the second drug delivery system included in the formulation at the start of administration or before administration.

Moreover, when the anti-inflammatory drug included in the first formulation causes an increase in the blood glucose level of the subject depending on the blood concentration of the anti-inflammatory drug, the concentration at which the increased level of blood glucose is acceptable may be an upper limit of the anti-inflammatory drug release amount, and the effective amount of preventing, alleviating or treating the inflammatory reaction caused by the second drug delivery system included in the second formulation may be set as a lower limit of the anti-inflammatory drug release amount, and the range of the anti-inflammatory drug release amount can be set appropriately within the combined range of the upper and lower limits.

In the present invention, the term "individual" or "subject" includes mammals, especially humans. The administration plan, administration interval, dosage, etc. are easily set, changed or adjusted by a person skilled in the art according to the above-mentioned factors.

In the present invention, when an anti-inflammatory first formulation containing an anti-inflammatory drug is used together with a second formulation including a drug being effective for preventing, alleviating or treating diseases, it is expected to suppresses the progression to chronic inflammation by reducing the inflammatory response caused by the drug delivery system of the second formulation at the site of administration, from the initial inflammation stage after administration, to improve the patient's reluctance or satisfaction by lowering side effects such as edema, erythema, and tissue hardening at the administration site, and to improve the therapeutic effect by single or repeated administration of the parenteral formulation including therapeutic agents for a period required for treatment.

In the present invention, the first formulation containing an anti-inflammatory drug at a concentration low enough to show anti-inflammatory efficacy locally at the site of inflammation induction, may maintain the continuous release of the anti-inflammatory drug until the second drug delivery system contained in the second formulation is completely decomposed, thereby providing a formulation capable of suppressing inflammation only at the administered site of the parenteral anti-inflammatory formulation, without the side effects caused by systemic exposure of the anti-inflammatory drug.

In the present specification, the first anti-inflammatory formulation may include only the anti-inflammatory drug, or may include the anti-inflammatory drug and the first drug delivery system. When the anti-inflammatory first formulation includes the anti-inflammatory drug and the first drug delivery system, the anti-inflammatory drug is contained in an amount of about 1% to about 90% by weight, about 2% to about 90%, from about 5% to about 90%, from 10% to about 90%, or from about 10% to about 80%, based on 100% by weight of the first drug delivery system included in the first formulation.

In the present specification, when the first drug and the second drug are loaded in one drug delivery system, for example microparticles, the first drug contained therein is about 0.001% by weight, 0.005% by weight, 0.01% by weight, 0.05% by weight, 0.1% by weight, 0.2% by weight, 0.5% by weight, 1% by weight, or 5% by weight, based on total amount of the drug delivery system.

In the present specification, "active ingredient", "active drug", "effective ingredient", "active agent", "drug" and "therapeutic agent" are used interchangeably herein, and are referred to any substance used to prevent, alleviate or treat a target disease.

In the present invention, the anti-inflammatory drug contained in the anti-inflammatory first formulation may be steroid preparations, compounds or drugs that prevents, alleviates or treats inflammation when administered at a pharmaceutically effective amount, that controls the degradation of the drug delivery system in co-administered second formulation, or that increases the bioavailability of the second drug used in combination, but not limited thereto.

More specifically, the anti-inflammatory drug may be referred to drugs for preventing, alleviating or treating an inflammatory reaction in a subject caused by administering to the subject a parenteral drug delivery system for delivering a drug for preventing, alleviating or treating a disease in the subject. For example, the anti-inflammatory drugs may be drugs for preventing, alleviating or treating an inflammatory reaction that occurs locally at the site of administration by a drug delivery system for parenteral delivery of an active ingredient for preventing, alleviating or treating a disease of the subject.

It is possible to use non-steroidal anti-inflammatory drugs and steroidal analgesics commonly used for the inflammation that has already occurred. When they are administered together with a sustained-release drug for treatment, steroidal anti-inflammatory drugs with strong anti-inflammatory effects are more preferably because they exhibit anti-inflammatory effects even at small amounts.

However, the high plasma level of steroidal anti-inflammatory drugs usually causes systemic side effects such as hypertension, hyperglycemia, osteoporosis, cataracts, glaucoma, and gastric ulcers, and when used for a long time, the drugs are no longer effective due to the drug resistance. When the steroidal anti-inflammatory drugs are administered by oral or intravenous injection as the conventional steroid formulation for a long period of time until the sustained-release injection formulation loaded with a therapeutic agent is decomposed in the body, they cannot be used for a long time due to the side effects of such systemic exposure.

Anti-inflammatory agents include, for example, non-steroidal anti-inflammatory agents, which include, but are not limited to, aspirin, diclofenac, flurbiprofen, ibuprofen, ketorolac, naproxen, and suprofen. In an embodiment, the anti-inflammatory agent includes a combination of two or more non-steroidal anti-inflammatory agents.

More specifically, nonsteroidal anti-inflammatory drugs include aceclofenac, acemetacin, alminoprofen, amfenac, apazone, aspirin, bromfenac, bufexamac, celecoxib, choline salicylate, cinnoxicam, clonixin, dexibuprofen, dexketoprofen, diclofenac, diflunisal, emorfazone, etodolac, etoricoxib, ethenzamide, felbinac, Fenoprofen, flufenamic acid, flurbiprofen, ibuprofen, imidazole salicylate, indomethacin, isopropylantipyrine, ketoprofen, ketorolac, lornoxicam, loxoprofen, meclofenamate, meloxicam, mefenamic acid, morniflumate, nabumetone, naproxen, nefopam, nimesulide, oxaprozin, oxyphenbutazone, pelubiprofen, phenylbutazone, piroxicam, pranoprofen, proglumetacin, rofecoxib, salsalate, salsalate salicylate, sulindac, talniflumate, tenoxicam, tiaprofenic acid, tolfenamic acid, tolmetin, valdecoxib, zaltoprofen, and pharmaceutically acceptable salts thereof, but not limited thereto.

Anti-inflammatory agents include drugs that prevent, reduce, or treat inflammation when administered in prophylactically or therapeutically effective amounts, and specifically include "steroidal anti-inflammatory agents," "corticoids," "corticosteroids," and "glucocorticosteroids" and the anti-inflammatory agents may include a combination of two or more steroidal anti-inflammatory agents.

The steroidal anti-inflammatory drugs that can be used in the present invention include 21- acetoxy pregnenolone, alclomethasone, algestone, amcinonide, beclomethasone, betamethasone, budesonide, chloroprednisone, and clobetasol. , clobetasone, clocortolone, cloprednol, corticosterone, cortisone, cortivazol, deflazacort, desonide, desoximethasone, dexamethasone, dexamethasone acetate, dexamethasone phosphate, diflorasone, diflucortolone, difluprednate, enoxolone, fluazacort, flucloronide, flumethasone, flunisolide, fluocinolone acetonide , fluocinonide, flucortin butyl, flucortolone, fluorometholone, fluperolone acetate, fluprednidene acetate, fluprednisolone, flurandrenolide, fluticasone propionate, formocortal, halcinonide, halobetasol propionate, halomethasone, halopredone acetate, hydrocortamate, hydrocortisone, lotepred loteprednol etabonate, mazipredone, medrysone, meprednisone, methylprednisolone, mometasone furoate, paramethasone, prednicarbate, prednisolone, prednisolone 25-diethylamino -acetate, prednisolone sodium phosphate, prednisone, prednival, prednylidene, rimexolone, tixocortol, triamcinolone, triamcinolone acetonide, triamcinolone venetonide, triamcinolone hexacetonide, and derivatives thereof, but limited thereto.

The corticosteroid is beclomethasone dipropionate, betamethasone, budesonide, deflazacort, dexamethasone, dexamethasone acetate, dexamethasone phosphate, difluprednate, epinephrine, fludrocortisone, fluocinolone acetonide, fluocortin, fluorometholone, fluticasone, hydrocortisone, methylprednisolone, prednisolone, prednisone, triamcinolone, and pharmaceutically acceptable salts thereof.

In the present specification, the second drug, which is an active ingredient included in the second formulation, may be, for example, a drug that is parenterally delivered as an active ingredient for preventing, alleviating or treating a disease or condition of a subject. The active ingredient that may be included in the second formulation, is different from the anti-inflammatory drug of the first anti-inflammatory formulation, and may be an active ingredient for preventing, alleviating or treating a disease or condition of a subject.

Specifically, the kinds of active ingredients that can be included in the second formulation are not particularly limited, but their examples can be selected from an agent for treating dementia; an agent for treating Parkinson's disease; anticancer agents; antipsychotic drugs such as anti-anxiety drugs, anti-depressants, tranquilizers and psychoactive agents; cardiovascular agents such as antihypertensive agents, antithrombotic agents, vasodilators, and antiarrhythmic agents; epilepsy medication; therapeutic agents for gastrointestinal such as anti-ulcer agents; rheumatic drugs; antispasmodic; agent for treating tuberculosis; muscle relaxants; agents for osteoporosis; drugs for erectile dysfunction; hemostatic agents; hormones such as sex hormones; antidiabetic agents; antibiotics; antifungal agents; antiviral agents; antipyretic analgesic and anti-inflammatory agents; autonomic nervous modulators; diuretic; antidiuretic; painkiller; antihistamines; antiprotozoal agents; anti-anemic agents; anti-asthmatics; antispasmodic; antidote; antimigraine drugs; anti-emetics; antiparkinsonian agents; antiepileptic drugs; antiplatelet agents; antitussive expectorants; bronchodilators; cardiotonic agents; immunomodulator; protein drugs; genetic drugs; and a mixture thereof, or preferably can be selected from drugs for treating dementia, drugs for treating Parkinson's disease, anticancer drugs, antipsychotic drugs, drugs for hyperlipidemia, drugs for hypertension, drugs for treating epilepsy, drugs for gastrointestinal disorders, drugs for rheumatism, antispasmodics, agents for treating tuberculosis, muscle relaxants, arrhythmia, an agent for osteoporosis, an erectile dysfunction agent, a hemostatic agent, an antiviral agent, a hormone agent, an antibiotic agent, an antidiabetic agent, an antifungal agent, an antithrombotic agent, an antipyretic analgesic and anti-inflammatory agent, and a mixture thereof.

In addition, the active ingredient that may be included in the second formulation may be a small molecule, protein, antibody, synthetic compound, nucleic acid molecule, or peptide. The nucleic acid molecule is at least one selected from the group consisting of DNA, RNA, microRNA (miRNA), small RNA (smRNA), small interfering RNA (siRNA), piRNA (Piwi-interacting RNA), small nucleolar RNA (snoRNA), t-RNA-derived small RNA (tsRNA), small rDNA-derived RNA (srRNA), small nuclear RNA (U-RNA) and long noncoding RNA (IncRNA).

It is not particularly limited among the above-mentioned drugs, but preferably donepezil, memantine, rivastigmine, entecavir, lamivudine, rotigotine, ropinirole, buprenorphine, fentanyl, nimodipine, and granisetron, cytarabine, carmustine, tamsoleucine, falmacoxib, testosterone, estradiol, risperidone, paliperidone, olanzapine, aripiprazole, goserelin, leuprolide, triptorelin, buserelin, naparelin, deslorelin, octreotide, pasireotide, lanreotide, barpretide, exenatide, liraglutide, lixisenatide, semaglutide, 5-alpha reductase inhibitors (e.g., finasteride, dutasteride, etc.), tirzepatide, dulaglutide, insulin glargine, insulin degludec, insulin icodec, cagrilintide and salts thereof, derivatives, and mixtures of two or more thereof.

The pharmaceutical kit for combined parenteral administration according to an embodiment of the present invention, may further include a local anesthetic component in addition to the anti-inflammatory first formulation and/or the second formulation. The local anesthetic component may be used as a drug together with the first formulation, the second formulation, or a mixed formulation of the first formulation and the second formulation, or may be used in an appropriately separate drug carrier.

Another embodiment of the present invention relates to a pharmaceutical kit, a formulation, or kit for combined parenteral administration that controls decomposition of a parenteral drug delivery system of a second formulation used in combination with an anti-inflammatory drug, or specifically, first formulation containing an inflammatory first drug, or an inflammatory first drug and a first parenteral drug delivery system, and a second formulation containing a second parenteral drug delivery system, wherein the first parenteral drug delivery system and the second parenteral drug delivery system contain biodegradable polymer,
wherein the first formulation or the drug included in the first formulation controls the decomposition rate of the second parenteral drug delivery system.

The second formulation may further include a pharmaceutically active ingredient being different from the anti-inflammatory drug of the first formulation, and the description of the second formulation is the same as described above.

The first formulation or the anti-inflammatory drug contained in the first formulation may control the dissolution rate of the second parenteral drug delivery system, thereby controlling the release rate of the drug contained in the second formulation. Controlling the decomposition rate of the second parenteral drug delivery system may preferably extend the drug release period by lowering the decomposition rate of the second parenteral drug delivery system.

The anti-inflammatory drug included in the first formulation is as described above, and may be, for example, dexamethasone free base, dexamethasone acetate, or dexamethasone phosphate, but is not limited thereto.

In addition, in the pharmaceutical kit for combined parenteral administration which controls the decomposition rate of the second parenteral drug delivery system, the first formulation and the second formulation may be included as a mixed preparation or separate preparation, or administered at the same time or at different times.

In the first formulation and the second formulation, the solid content of first formulation may 0.002 to 20 parts by weight based on 100 parts by weight of the solid content of the second formulation, and the second formulation may or may not contain a drug.

When the first formulation and the second formulation are provided as a mixed formulation, the anti-inflammatory drug may be included in an amount of 0.001 to 5.0 w/w% based on 100% by weight of the solid content of the mixed formulation.

In the present specification, the first formulation includes an anti-inflammatory drug and a first drug delivery system, a second formulation includes drug and a second drug delivery system, and the first formulation and the second formulation are provided as a mixed formulation. In this case, the first drug delivery system may be included in 0.002 to 20% by weight w/w%, based on 100% by weight of the solid content of the mixed formulation.

A pharmaceutical kit for parenteral use according to an embodiment of the present invention includes a first formulation containing a first drug and a first parenteral drug delivery system, and a second parenteral formulation containing a pharmaceutically active ingredient different from the first drug in the first formulation, and a second parenteral drug delivery system. In this case, the first drug contained in the first formulation may be released during the same period as the administration interval of the second formulation or during a part of the administration interval of the second formulation, so as to achieve the desired efficacy. In another example, the first drug included in the first formulation is uniformly released at a constant concentration during the release period. Alternatively, at a certain time depending on the characteristics required by the second formulation, for example, during the initial period or the later period of 50% or less, 40% or less, 30% or less, 20% or less, or 10% or less of the entire administration interval of the second formulation, the first drug may be released at a relatively high or low concentration to have nonuniform release characteristics. In another example, the first drug contained in the first formulation may be released at a concentration which is proportional to the remaining amount of the second parenteral drug delivery system contained in the second formulation. Depending on the relative content (wt %) of the second drug delivery system remaining at the administration site on the basis of 100% by weight of the content of the second drug delivery system at the starting point of administration or before administration, the drug in the first formulation may be released at a gradually decreased amount.

Moreover, when the anti-inflammatory drug included in the first formulation causes an increase in the blood glucose level of a subject depending on the blood concentration of drug, the drug concentration at which the increased level of blood glucose is acceptable may be the upper limit of the anti-inflammatory drug release amount, and the effective amount of preventing, alleviating, or treating the inflammatory reaction caused by the second drug delivery system included in the second formulation may be set as the lower limit of the anti-inflammatory drug release amount, and the range of the anti-inflammatory drug release amount can be set appropriately within the combined ranges of the upper and lower limits.

In addition, the material, formulation, or characteristics of the drug delivery system are as described above, and the active ingredients that can be included in the second formulation are also described above.

An embodiment of the present invention relates to a pharmaceutical kit for combined parenteral administration, comprising a first formulation containing a first drug, and a second formulation including a second drug and a parenteral drug delivery system, wherein the first formulation includes a first anti-inflammatory drug or a first anti-inflammatory drug loaded in a first parenteral drug delivery system, wherein the second formulation contains a second parenteral drug delivery system and a second drug that is different from the drug of the first formulation, and wherein the first formulation is for increasing bioavailability of the second drug included in the second formulation.

In the present invention, the first anti-inflammatory drug or the first anti-inflammatory drug loaded in a first parenteral drug delivery system, is an agent for regulating or enhancing bioavailability of the second drug.

As used herein, the term "bioavailability" refers to the dosage fraction of unchanged drug that reaches the systemic circulation, and is one of the major pharmacokinetic properties of a drug. When the drug is administered intravenously, its bioavailability is 100%. When a drug is administered by other routes (e.g., orally), its bioavailability is generally reduced or may vary from patient to patient. Bioavailability is an important parameter of pharmacokinetics that is considered for calculating drug dosage for non-intravenous administration route.

The pharmaceutical kit for combined parenteral administration of the present invention requires a smaller dose of drug and improves the pharmacokinetic profile and efficacy of the second drug, compared to second formulation alone without combining with the first formulation containing the first drug.

Due to the improved bioavailability according to the present invention, a drug previously used for intramuscular injection or the like can be changed to a subcutaneously injectable formulation, or formulated with a lower content of drug. That is, lower frequency or lower dosage of administration can reduce toxicity-related side effects. In general, the subcutaneous formulation has an improved pharmacokinetics and/or pharmacodynamics, and has advantages over conventional intramuscular injection. For example, subcutaneous injection can be performed at home or anywhere by allowing injection by a patient or family member. Due to the improved bioavailability of the present invention, there are advantages such as change in the administration route of the second formulation, dosage reduction, and/or reduced frequency of administration. In addition, due to improved bioavailability, it is possible to reduce the effective amount of the drug, or the content of the drug loaded in a unit dosage form, to change the intramuscular injection into other administration route such as subcutaneous injection, and to reduce the frequency of administration, so as to improve drug compliance of patient.

The term "effective amount" refers to that amount of a therapeutic compound effective at the time of administration to achieve the desired therapeutic or prophylactic result in the required period of time. The term, "therapeutically effective amount" of a therapeutic compound may vary depending on factors such as the disease state, age, sex, and body weight of the individual, and the ability of the drug to elicit a desired response in the individual.

In a specific embodiment, the use of increasing, enhancing, or improving the bioavailability of the second drug according to the present invention includes an increase in Cmax and/or AUC. For example, compared to a single formulation of the second formulation without the first formulation, a pharmaceutical kit for combined administration containing the first formulation and the second formulation refers to an increase in Cmax and/or AUC of the second drug. More specifically, compared to a single formulation of the second formulation, the pharmaceutical kit for combined administration including the first formulation and the same second formulation, has Cmax of 1.1 times or more, 1.2 times or more, 1.3 times or more, 1.4 times or more, 1.5 times or more, 2.0 times or more, and/or AUC of 120% or more, 130% or more, 150% or more, 200% or more, 250% or more, or 300% or more.

The material, dosage form, or characteristics of the drug delivery system are as described above, and the active ingredients that can be included in the second formulation are also described above. In addition, the use of improving the bioavailability in the present invention relates to the improved bioavailability of the second drug included in the second formulation described above, and specific examples of the second drug are donepezil, rivastigmine, semaglutide, leuprolide, octreotide, finasteride, and the like, but are not intended to be limited thereto.

A specific embodiment of the present invention relates to a pharmaceutical kit for combined parenteral administration that improves bioavailability of a co-administered drug (a drug of a second formulation) by using an anti-inflammatory drug of the first formulation.

A parenteral preparation containing a drug together with a biodegradable polymer for long-term dissolution, for example, a microsphere preparation, has been known. However, as the drug encapsulated in the microsphere has a low bioavailability, it should be a large amount of microspheres in order to achieve effective efficacy for a long time. When the large amount of microspheres are administered into the body, it is difficult to inject subcutaneously, resulting in difficulty of making for a patient to administer them by himself (self-administration), or a problem of greatly increased pain and inflammatory reaction at the site of administration. Therefore, there is provided a pharmaceutical kit for parenteral combined administration including the first formulation and the second formulation containing a drug or a pharmaceutically acceptable salt thereof and a biodegradable polymer, where the kit improves bioavailability of the drug and exhibits stable drug release characteristics for a long period of time.

Specifically, an embodiment of the present invention may be a pharmaceutical kit for combined parenteral administration including a first formulation containing a first drug, and a second formulation containing a second drug or a pharmaceutically acceptable salt thereof and a parenteral drug delivery system where the first formulation includes a first anti-inflammatory drug or a first anti-inflammatory drug loaded in a first parenteral drug delivery system, and where the second formulation contains a second parenteral drug delivery system and a drug or a pharmaceutically acceptable salt thereof as a drug in the second formulation, wherein the first formulation is for increasing or improving the bioavailability of the drug contained in the second formulation. In the present invention, the first formulation containing the first anti-inflammatory drug or the first anti-inflammatory drug loaded in the first parenteral drug delivery system is a bioavailability modifier or enhancer of the second drug.

When the first drug included in the pharmaceutical kit is provided without an individual drug delivery system, the first drug can be provided in the form of a mixed formulation of the second formulation containing a second parenteral drug delivery system and a second drug, or the first drug can be provided separately from the second formulation at different times. Specifically, when the second formulation is microparticles, the first drug and the second drug may be provided in a form supported together in one microparticle.

Alternatively, the first drug included in the pharmaceutical kit may be provided in a form supported in a separate first drug delivery system, and the first drug delivery system may be the same as or different from the second drug delivery system. Specifically, when the first formulation includes the first drug loaded in the first parenteral drug delivery system, it may be provided in the form of a mixed formulation or separate formulations with the second formulation, and can be administered to a subject in need thereof at a same time or different times.

In the embodiment, the first formulation, the first drug contained in the first formulation, the first parenteral drug delivery system that may be included in the first formulation, and/or the second parenteral drug delivery system included in the second formulation are described above. In addition, the bioavailability, the bioavailability modifier or enhancer of the second drug, and the like are described above.

The pharmaceutical kit for combined parenteral administration according to the present invention may be administered parenterally, for example, through subcutaneous injection. The second formulation may be composed of a drug unit and a solvent unit used to suspend the drug, and can be a dual chamber syringe containing a drug unit in one chamber and a solvent unit in another chamber, or a pre-filled syringe in which the drug unit is suspended in the solvent unit. When the drug unit is configured in the form of a prefilled syringe in which the drug unit is suspended in the solvent unit, the used solvent may be injectable oils including medium chain oil, mineral oil, and the like.

An example of a drug being co-administered in the kit may be an active ingredient to be included in the second formulation described above, and specifically can be selected from semaglutide, donepezil, rivastigmine, finasteride, octreotide, leuprolide, deslorelin, entecavir, and salts and derivatives thereof, and mixtures of two or more thereof.

For example, the pharmaceutically acceptable salt of semaglutide may be salts of sodium, acetate, benzoate, hydroxynaphthoate, naphadisylate or pamoate. The pharmaceutically acceptable salt may be leuprolide acetate, donepezil hydrochloride as salt of donepezil, rivastigmine tartrate and rivastigmine pamoate as pharmaceutically acceptable salt of rivastigmine, octreotide acetate, deslorelin acetate, and the like.

The anti-inflammatory drug of the first formulation included in the kit may be an active ingredient to be included in the first formulation described above, or specifically can be selected from dexamethasone, meloxicam, ketorolac, salts and derivatives thereof, and mixtures of two or more thereof. In particular, the dexamethasone and its derivatives can be dexamethasone, dexamethasone acetate, dexamethasone phosphate, and the like, but not limited thereto.

The pharmaceutical kit for combined parenteral administration comprising a second drug and/or a pharmaceutically acceptable salt thereof according to an embodiment of the present invention, may include a high content of the second drug compared to the particle size, and has high bioavailability when administered into the body, thereby reducing the unit dose with long-lasting effects. Therefore, it has the advantage of minimizing pain and inflammatory reaction of patient that may occur at administration.

The pharmaceutical kit for combined parenteral administration including the second drug and/or its pharmaceutically acceptable salt may include a high content of the second drug compared to the content of microsphere, suppresses an initial excessive release of the drug that can cause fatal side effects, and has high bioavailability, thereby improving, preventing, or treating diseases by showing sufficient drug efficacy for a desired period of time.

In addition, the increased bioavailability of the second drug according to the present invention can maintain an effective concentration of drug even near end of unit administration interval, which is very excellent in terms of maintaining the drug concentration through the drug administration interval. For example, when the second drug is leuprolide, the increased bioavailability of the present invention solves the inflammatory problem caused by the administration of the second drug, and improves the bioavailability of the second drug, especially at the end point of the unit administration interval. That is, the increased bioavailability can improve side effects induced by a sharp increase (surge) in testosterone levels which is caused by a decrease in drug concentration during a certain period of time before and after the start point of the subsequent administration.

In a specific embodiment, the use of increasing, improving, or enhancing the bioavailability of the second drug according to the present invention includes an increase in Cmax and/or AUC. For example, compared a second formulation alone without the first formulation, a pharmaceutical kit for combined administration containing the first formulation and the second formulation is referred to mean an increase in Cmax and/or AUC of the second drug. More specifically, compared to a single formulation of the second formulation, the pharmaceutical kit for combined administration including the same second formulation as the single formulation and the first formulation, has Cmax of 1.1 times or more, 1.2 times or more, 1.3 times or more, 1.4 times or more, 1.5 times or more, or 2.0 times or more, and/or AUC of 120% or more, 130% or more, 150% or more, 200% or more, 250% or more, or 300% or more.

More particularly, when the second drug is semaglutide, it can be useful for prevention or treatment of diabetes, specifically type 2 diabetes, preservation of beta-cell function, hypertension, hyperlipidemia, obesity, non-alcoholic steatohepatitis or degenerative neurological diseases such as Alzheimer's disease and Parkinson's disease. In the pharmaceutical kit for combined parenteral administration including the semaglutide and/or its pharmaceutically acceptable salt, based on 100% by weight of the total second formulation including the second drug and the second parenteral drug delivery system, the content of the second drug may be 5% by weight or more, 8% by weight or more, 9% by weight or more, 9.5% by weight or more, or 10% by weight or more, for example, 5 to 20% by weight. In addition, in a pharmaceutical kit for combined parenteral administration, a unit dose of the second formulation (e.g., microspheres containing a biocompatible polymer) including semaglutide and/or a pharmaceutically acceptable salt thereof and a parenteral drug delivery system, may be 10 to 800 mg.

As an example of the present invention, when the anti-inflammatory drug of the first formulation is dexamethasone, a derivative thereof or a salt thereof, and the drug of the second formulation is semaglutide or a salt thereof, the weight ratio of the first drug, dexamethasone (free base) to the second drug, semaglutide (free base) in the pharmaceutical kit for combined parenteral administration may range from 0.0001:1 to 1:1. It may preferably range from 0.004:1 to 0.2:1. More preferably, it may range from 0.001:1 to 0.05:1.

As an example of the present invention, when the anti-inflammatory drug of the first formulation is dexamethasone, a derivative thereof or a salt thereof, and the drug of the second formulation is donepezil or a salt thereof, the weight ratio of the first drug, dexamethasone (free base) to the second drug, donepezil (free base) in the pharmaceutical kit for combined parenteral administration may range from 0.00003:1 to 0.02:1. It may preferably range from 0.00006:1 to 0.001:1. More preferably, it may range from 0.0001:1 to 0.005:1.

When the second drug is donepezil, the content of the second drug in the pharmaceutical kit for combined parenteral administration may be 25 to 75% by weight or 27 to 75% by weight based on 100% by weight of the total second formulation including the second drug and the second parenteral drug delivery system. In addition, in the pharmaceutical kit for combined parenteral administration, the unit dose of the second formulation (e.g., microspheres containing a biocompatible polymer) containing the donepezil and/or a pharmaceutically acceptable salt thereof and a parenteral drug delivery system may be 50 to 1,200 mg.

As an example of the present invention, when the anti-inflammatory drug of the first formulation is dexamethasone, a derivative thereof, or a salt thereof, and the drug of the second formulation is rivastigmine or a salt thereof, the weight ratio of the first drug, dexamethasone (free base) to the second drug, rivastigmine (free base) in the pharmaceutical kit for combined parenteral administration may range from 0.0005:1 to 0.2:1. It may preferably range from 0.0001:1 to 0.1:1. More preferably, it may range from 0.00015:1 to 0.05:1.

When the second drug is rivastigmine, the content of the second drug in the pharmaceutical kit for combined parenteral administration may be 15 to 45% by weight or 20 to 40% by weight based on 100% by weight of the total second formulation including the second drug and the second parenteral drug delivery system. In addition, in the pharmaceutical kit for combined parenteral administration, the unit dose of the second formulation (e.g., microspheres containing a biocompatible polymer) containing the rivastigmine and/or a pharmaceutically acceptable salt thereof and a parenteral drug delivery system may be 20 to 900 mg.

When the second drug is finasteride, the content of the second drug in the pharmaceutical kit for combined parenteral administration may be 20 to 75% by weight or 30 to 70% by weight based on 100% by weight of the total second drug including the second drug and the second parenteral drug delivery system. In addition, in the pharmaceutical kit for combined parenteral administration, the unit dose of the second formulation (e.g., microspheres containing biocompatible polymers) containing the finasteride and/or its pharmaceutically acceptable salt and a parenteral drug delivery system may be 15 to 450 mg or 20 to 300 mg.

As an example of the present invention, when the anti-inflammatory drug of the first formulation is dexamethasone, a derivative thereof, or a salt thereof, and the drug of the second formulation is finasteride or a salt thereof, the weight ratio of the first drug, dexamethasone (free base) to the second drug, finasteride (free base) in the pharmaceutical kit for combined parenteral administration may range from 0.0003:1 to 0.3:1. It may preferably range from 0.0006:1 to 0.15:1. More preferably, it may range from 0.0009: 1 to 0.1:1.

When the second drug is octreotide, the content of the second drug in the pharmaceutical kit for combined parenteral administration may be 15 to 45% by weight or 20 to 40% by weight, based on 100% by weight of the total second drug including the second drug and the second parenteral drug delivery system. In addition, in the pharmaceutical kit for combined parenteral administration, the unit dose of the second formulation (e.g., microspheres containing biocompatible polymers) containing octreotide and/or its pharmaceutically acceptable salt and a parenteral drug delivery system may be 20 to 300 mg or 20 to 600 mg.

As an example of the present invention, when the anti-inflammatory drug of the first formulation is dexamethasone, a derivative thereof, or a salt thereof, and the drug of the second formulation is octreotide or a salt thereof, the weight ratio of the first drug, dexamethasone (free base) to the second drug, octreotide (free base) in the pharmaceutical kit for combined parenteral administration may range from 0.0003:1 to 0.6:1. It may preferably range from 0.0006:1 to 0.3:1. More preferably, it may be in the range of 0.0009:1 to 0.15: 1.

When the second drug is leuprolide, the content of the second drug in the pharmaceutical kit for combined parenteral administration may be 5 to 25% by weight or 10 to 20% by weight, based on 100% by weight of the total second drug including the second drug and the second parenteral drug delivery system. In addition, in the pharmaceutical kit for combined parenteral administration, the unit dose of the second formulation (e.g., microspheres containing biocompatible polymers) containing leuprolide and/or its pharmaceutically acceptable salt and a parenteral drug delivery system may be 15 to 230 mg or 18 to 225 mg.

As an example of the present invention, when the anti-inflammatory drug of the first formulation is dexamethasone, a derivative thereof, or a salt thereof, and the drug of the second formulation is leuprolide or a salt thereof, the weight ratio of the first drug, dexamethasone (free base) to the second drug, leuprolide (free base) in the pharmaceutical kit for combined parenteral administration may range from 0.0001: 1 to 0.6:1. It may preferably range from 0.0002:1 to 0.3:1. More preferably, it may be in the range of 0.0003:1 to 0.15:1.

When the second drug is entecavir, the content of the second drug in the pharmaceutical kit for combined parenteral administration may be 15 to 35% by weight or 20 to 35% by weight, based on 100% by weight of the total second drug including the second drug and the second parenteral drug delivery system. In addition, in the pharmaceutical kit for combined parenteral administration, the unit dose of the second formulation (e.g., microspheres containing biocompatible polymers) containing entecavir and/or its pharmaceutically acceptable salt and a parenteral drug delivery system may be 45 to 1200 mg.

As an example of the present invention, when the anti-inflammatory drug of the first formulation is dexamethasone, a derivative thereof, or a salt thereof, and the drug of the second formulation is entecavir or a salt thereof, the weight ratio of the first drug, dexamethasone (free base) to the second drug, entecavir (free base) in the pharmaceutical kit for combined parenteral administration may range from 0.00015:1 to 0.2:1. It may preferably range from 0.0003:1 to 0.1:1. More preferably, it may range from 0.0006:1 to 0.05:1.

In an embodiment according to the present invention, when the first drug delivery system included in the first anti-inflammatory formulation is microspheres, or the second drug delivery system included in the second formulation is microspheres, it may be prepared using a method for producing microparticles well known in the art, for example, a solvent extraction and evaporation method or a spray drying method, but is not limited thereto. Preferably, the sustained-release microspheres of dexamethasone according to the present invention can be prepared by using, for example, an O/W method, a W/O/W method, or a S/O/W method.

When the first formulation according to the present invention is a microsphere containing an anti-inflammatory drug, the preparation method thereof can be performed by S/O/W method, specifically (a1) preparing a disperse phase solution by homogeneously dispersing an anti-inflammatory drug, for example, dexamethasone particles in a solution in which a biocompatible polymer is dissolved, (b1) preparing an emulsion by adding the disperse phase solution prepared in step (a1) to an aqueous phase solution (continuous phase) containing a surfactant, (c1) forming microspheres by extracting an organic solvent from the disperse phase solution in the emulsion state prepared in step (b1) into the continuous phase solution and evaporating the organic solvent, and (d1) preparing sustained-release microspheres containing dexamethasone by recovering the microspheres from the continuous phase solution of step (c1).

When the first formulation according to the present invention is a microsphere containing an anti-inflammatory drug, the preparation method thereof can be performed by W/O/W method, specifically (a2) preparing W1 phase solution by dissolving an anti-inflammatory drug, for example dexamethasone in distilled water, preparing oil phase solution by dissolving a biocompatible polymer in a poorly water-soluble organic solvent, and preparing a primary emulsion by homogeneously dispersing the W1 phase solution in the oil phase, (b2) preparing an emulsion by adding the disperse phase solution prepared in step (a2) to an aqueous phase solution (continuous phase) containing a surfactant, (c2) forming microspheres by extracting the organic solvent from the disperse phase solution in the emulsion state prepared in step (b) into the continuous phase solution and evaporating the organic solvent, and (d2) preparing sustained-release microspheres containing dexamethasone by recovering microspheres from the continuous phase solution of step (c2).

When the first formulation according to the present invention is a microsphere containing an anti-inflammatory drug, the preparation method thereof can be performed by O/W method, specifically (a3) preparing disperse phase solution by dissolving an anti-inflammatory drug, for example dexamethasone and a biocompatible polymer in an organic solvent, (b3) preparing an emulsion by adding the disperse phase solution prepared in step (a3) to an aqueous phase solution (continuous phase) containing a surfactant, (c3) forming microspheres by extracting the organic solvent from the disperse phase solution in the emulsion state prepared in step (b3) into the continuous phase solution and evaporating the organic solvent, and (d3) preparing sustained-release microspheres containing dexamethasone by recovering microspheres from the continuous phase solution of step (c3).

In the method for preparing dexamethasone microspheres according to the present invention, the biocompatible polymer or biodegradable polymer in step (a) may be a biodegradable polymer having as an intrinsic viscosity of 0.16 to 1.9 dL/g or 0.10 to 1.3 dL/g, preferably 0.16 dl/g to 0.75 dL/g, in consideration of factors such as drug release characteristics and manufacturing process, etc. The intrinsic viscosity refers to a value measured at a concentration of 0.1% (w/v) in chloroform at 25 °C using an Ubbelohde viscometer.

In an embodiment according to the present invention, when the second drug delivery system included in the second formulation is microspheres, it may be prepared using a method for producing microparticles well known in the art, for example, a solvent extraction and evaporation method or a spray drying method, but is not limited thereto. Preferably, the drug microspheres of second formulation according to the present invention can be prepared by using, for example, O/W method, W/O/W method or S/O/W method.

The drug microsphere of the second formulation, the preparation method thereof can be prepared by S/O/W method, specifically (a4) preparing a disperse phase solution by homogeneously dispersing drug particles in a solution in which a biocompatible polymer is dissolved, (b4) preparing an emulsion by adding the disperse phase solution prepared in step (a4) to an aqueous phase solution (continuous phase) containing a surfactant, (c4) forming microspheres by extracting the organic solvent from the disperse phase solution in the emulsion state prepared in step (b4) into the continuous phase solution and evaporating the organic solvent, and (d4) preparing sustained-release microspheres containing the drug in the second formulation by recovering microspheres from the continuous phase solution of step (c4).

The drug microsphere of the second formulation, the preparation method thereof can be prepared by W/O/W method, specifically (a5) preparing W1 phase solution by dissolving a drug, in distilled water, preparing oil phase solution by dissolving a biocompatible polymer in a poorly water-soluble organic solvent, and preparing a primary emulsion by homogeneously dispersing the W1 phase solution in the oil phase solution, (b5) preparing an emulsion by adding the disperse phase solution prepared in step (a5) to an aqueous phase solution (continuous phase) containing a surfactant, (c5) forming microspheres by extracting the organic solvent from the disperse phase solution in the emulsion state prepared in step (b5) into the continuous phase solution and evaporating the organic solvent, and (d5) preparing sustained-release microspheres containing the drug in the second formulation by recovering microspheres from the continuous phase solution of step (c5).

The drug microsphere of the second formulation, the preparation method thereof can be prepared by O/W method, specifically (a6) preparing disperse phase solution by dissolving a drug and a biocompatible polymer in an organic solvent, (b6) preparing an emulsion by adding the disperse phase solution prepared in step (a6) to an aqueous phase solution (continuous phase) containing a surfactant, (c6) forming microspheres by extracting the organic solvent from the disperse phase solution in the emulsion state prepared in step (b6) into the continuous phase solution and evaporating the organic solvent, and (d6) preparing sustained-release microspheres containing the drug in the second formulation by recovering microspheres from the continuous phase solution of step (c6).

In addition, in one example according to the present invention, the first drug of the first formulation and the second drug of the second formulation can be loaded together in one drug delivery system, specifically the second drug delivery system, and the second drug delivery system is microspheres. In this case, the method for preparing the microspheres may also be applied to the method for preparing the microspheres of the first formulation or the microspheres of the second formulation.

In an embodiment according to the present invention, when the mixed formulation of the second formulation containing the second drug and the second parenteral drug delivery system together with the anti-inflammatory first drug are microspheres, it may be prepared by substantially the same method as that of the second drug delivery system, but the first anti-inflammatory drug is included in the disperse phase solution together with the second drug in the step of preparing the disperse phase solution. In a specific embodiment, the disperse phase solution can be prepared by dissolving leuprolide acetate as the second drug and dexamethasone acetate as the first drug in dichloromethane and methyl alcohol, and can be added to a continuous phase solution containing a surfactant to prepare and lyophilize microspheres containing leuprolide and dexamethasone acetate.

Specifically, the method for producing microspheres according to the present invention includes a step of preparing an emulsion by adding the disperse phase prepared in step (a) to an aqueous phase solution (continuous phase) containing a surfactant.

In the method for preparing drug microspheres according to the present invention, the biocompatible polymer or biodegradable polymer in step (a) may be a biodegradable polymer having as an intrinsic viscosity of 0.16 to 1.9 dL/g or 0.10 to 1.3 dL/g, preferably 0.16 dL/g to 0.75 dL/g, in consideration of factors such as drug release characteristics and manufacturing process, etc. The intrinsic viscosity refers to a value measured at a concentration of 0.1% (w/v) in chloroform at 25 °C using an Ubbelohde viscometer.

In the step (b), the content of surfactant in the continuous phase solution containing the surfactant may be 0.01% to 20% by weight, or preferably 0.1% to 5% by weight, based on the total volume of the continuous phase solution containing the surfactant. When the content of surfactant is less than 0.01% by weight, the droplet forms of the disperse phase solution or emulsion may not be formed in the continuous phase solution, and when the content of surfactant exceeds 20% by weight, there may be difficulty in removing the surfactant after particle formation due to the excessive surfactant.

In the step (b), the method of homogeneously mixing the biodegradable polymer solution in which the drug is disperse, and the continuous phase solution containing the surfactant is not particularly limited, but can be performed using a high-speed stirrer, an in-line mixer, an ultrasonic disperser, a static mixer, a membrane emulsion method, a microfluidics emulsion method and the like. When forming an emulsion with a high-speed stirrer, an in-line mixer, an ultrasonic disperser, or a static mixer, the method can further include preferably a step of an additional particle size screening process between steps (c) and (d) described below, because it is difficult to obtain a uniform emulsion.

Sodium chloride may be added to the continuous phase solution containing the surfactant in order to prevent loss of the drug in the emulsion formed in the step (b).

The method for preparing drug microspheres according to the present invention includes the steps of c) forming microspheres by extracting the organic solvent from the disperse phase solution in the emulsion state prepared in step (b) into the continuous phase solution and evaporating the organic solvent, and (d) preparing sustained-release microspheres containing the first drug and/or the second drug by recovering microspheres from the continuous phase solution of step (c).

In the step (c), when the emulsion including the disperse phase solution in the form of droplets and the continuous phase solution containing the surfactant is maintained or stirred at a temperature below the boiling point of the organic solvent for a certain period of time, for example, from 2 hours to 48 hours, the organic solvent can be extracted to the continuous phase solution from the disperse phase solution in the form of droplets including biocompatible polymer and drug. A part of the organic solvent extracted into the continuous phase solution may evaporate from the surface of emulsion. As the organic solvent is extracted and evaporated from the droplet forms of biocompatible polymer solution in which the drug is dispersed, the disperse phase solution in the form of droplets is solidified to form microspheres.

In the step (c), the temperature of the continuous phase solution may be heated for a certain period of time in order to efficiently remove the organic solvent.

In the step (c), ethanol may be added to the continuous phase solution to efficiently remove the organic solvent.

In the step (c), the surface of the microspheres may be modified by additionally adjusting the temperature of the continuous phase solution in order to control an initial release of the drug from the sustained-release microspheres. For this purpose, when the temperature is controlled by applying heat to the continuous phase solution, the lower limit of the temperature range may be 30 °C or the glass transition temperature of the polymer, and the upper limit may be 30 °C higher than the glass transition temperature of the biocompatible polymer (Tg of polymer + 30 °C). It can be adjusted within the numerical range by the combination of the lower limit and the upper limit.

In the step (d), the method of recovering the sustained-release microspheres can be performed using various known techniques, such as filtration or centrifugation.

Between the step (c) and the step (d), the microspheres may be recovered by filtering and washing them to remove the remaining surfactant, and filtering again. The washing step for removing the remaining surfactant may be typically performed using water, and the washing step may be repeated several times.

In the method for preparing sustained-release microspheres containing a drug according to the present invention, after the step (d) or after the filtration and washing steps, the obtained microspheres are dried by a conventional drying method to finally obtain dried microspheres.

When the first formulation and/or the second formulation are microsphere formulations, the same or different polymers may be used, and the weight average molecular weight of the applicable biodegradable polymer is not particularly limited, but the lower limit thereof may be 5,000 or more, or preferably 10,000 or more, and the upper limit may be 500,000 or less, or preferably 200,000 or less.

The kinds of the biodegradable polymers are not particularly limited and are as described above. For example, the biodegradable polymer may include at least one selected from the group consisting of a polyethylene glycol-poly(lactide-co-glycolide) block-copolymer, a polyethylene glycol-polylactide block-copolymer, polyethylene glycol-polycaprolactone block-copolymer, polylactide, polyglycolide, poly(lactide-co-glycolide), poly(lactide-co-glycolide)glucose, polycaprolactone, and mixtures thereof, and specifically, polylactide, poly(lactide-co-glycolide) and polycaprolactone may be used. When poly(lactide-co-glycolide) is used as the biodegradable polymer, the molar ratio of lactic acid to glycolic acid in the copolymer may be 99:1 to 50:50, or preferably 50:50, 75 :25 or 85:15.

When two or more kinds of biodegradable polymers are included, they may be combinations or blends of different polymers selected from the exemplified polymers, and they may be combinations of the polymers which belong to the same kind but have different intrinsic viscosities and/or monomer ratios. (e.g., combinations or blends of two or more poly(lactide-co-glycolide) with different intrinsic viscosities), or may be polymers which belong to the same kind of polymer but have different terminal groups (e.g., ester end groups or acid end groups).

Examples of commercially available biodegradable polymers that can be used in the present invention, include Evonik's Resomer series of RG502H, RG503H, RG504H, RG502, RG503, RG504, RG653H, RG752H, RG752S, 753H, 753S, RG755S, RG756S, RG858S, R202H, R203H, R205H, R202S, R203S, R205S; and Corbion's PDL 02A, PDL 02, PDL 04, PDL 05, PDLG 7502A, PDLG 7502, PDLG 7504A, PDLG 7504, PDLG 7507, PDLG 5002A, PDLG 5002, PDLG 5004A , PDLG 5004, PDLG 5010, PL 10, PL 18, PL 24, PL 32, PL 38, PDL 20, PDL 45, PC 02, PC 04, PC 12, PC 17, PC 24, etc. alone or in combination or blends thereof, but are not limited thereto. A suitable molecular weight or blending ratio of the biodegradable polymers can be appropriately selected by a person skilled in the art in consideration of the decomposition rate of the biodegradable polymer and the resulting drug release rate. In a specific embodiment, to prepare the microparticles according to the present invention, Resomer R755S (iv = 0.50-0.70 dL/g; manufacturer: Evonik, Germany) or Resomer R752H (iv = 0.16-0.24 dL/g; manufacturer: Evonik, Germany) can be used. Alternatively, as the specific polymer, Resomer R 205S (iv = 0.55-0.75 dL/g; manufacturer: Evonik, Germany) or Purasorb PDLG 7502A (iv = 0.16-0.24 dL/g; manufacturer: Purac, Netherlands) may be used.

### [Effects of the Invention]

The present invention provides a parenteral anti-inflammatory formulation for preventing, alleviating g or treating an inflammatory reaction caused by a pharmaceutical product administered parenterally to body, a parenteral formulation or kit for controlling the release rate of a formulation containing a parenteral second drug delivery system, or a parenteral formulation or kit containing a drug capable of increasing a bioavailability of the second drug.

### [Brief Description of Drawings]

Fig. 1 is a graph showing the improvement in bioavailability of a formulation containing leuprolide according to an example of Example 2.
Fig. 2 is a photograph showing the degree of infiltration, angiogenesis, and fibrous tissue formation of inflammatory cells by subcutaneously injecting placebo microspheres including no drug into experimental animals, and analyzing inflammation at the administration site of microsphere with an optical microscope after H&E (hematoxylin & eosin) staining.
Figs. 3a and 3b are photographs showing the degree of infiltration, angiogenesis, and fibrous tissue formation of inflammatory cells by subcutaneously injecting anti-inflammatory dexamethasone microspheres into experimental animals, and analyzing inflammation at the microsphere administration site with an optical microscope after H&E (hematoxylin & eosin) staining.
Figs. 4a, 4b, and 4c are pharmacokinetic graphs showing drug release over time in the blood by subcutaneously injecting anti-inflammatory microspheres as a first formulation into experimental animals.
Figs. 5a and 5b are photographs showing the degree of infiltration, angiogenesis, and fibrous tissue formation of inflammatory cells by subcutaneously injecting anti-inflammatory dexamethasone microspheres and placebo microspheres including no drug, into experimental animals, and analyzing inflammation at the microsphere administration site with an optical microscope after H&E (hematoxylin & eosin) staining.
Fig. 6 is a photograph showing the degree of inflammatory cell infiltration, angiogenesis, and fibrous tissue formation by subcutaneously injecting anti-inflammatory microspheres and leuprolide microspheres into experimental animals, and analyzing inflammation at the microsphere administration site with an optical microscope after H&E (hematoxylin & eosin) staining.
Fig. 7 shows the results showing the infiltration of inflammatory cells by preparing histopathology slide specimens of experimental groups G15 to G20.
Fig. 8 shows the result of showing the concentration of TGF-β2 in blood as an index for systemic inflammatory response by ELISA method.
Fig. 9 is a photograph showing residual microspheres at the administration site after administering placebo microspheres alone or a mixture of placebo microspheres and anti-inflammatory microspheres into experimental animals.
Fig. 10 is a photograph showing residual microspheres at the administration site after administration of leuprolide microspheres alone or a mixture of leuprolide microspheres and dexamethasone microspheres into experimental animals.
Fig. 11 is a graph showing blood concentrations of donepezil in blood collected from experimental animals and measured using LC-MS/MS.
Fig. 12 is a graph showing the blood concentration of rivastigmine in blood collected from experimental animals and measured using LC-MS/MS.
Fig. 13 is a graph showing the blood concentration of finasteride in blood collected from experimental animals and measured using LC-MS/MS.
Fig. 14 is a graph showing the blood concentration of semaglutide in blood collected from experimental animals and measured using LC-MS/MS.
Fig. 15 is a graph showing the blood concentration of octreotide in blood collected from experimental animals and measured using LC-MS/MS.
Fig. 16 is a graph showing the blood concentration of leuprolide in blood collected from experimental animals and measured using LC-MS/MS.
Fig. 17 is a graph showing the change in blood concentration of dexamethasone according to co-administration of dexamethasone base and dexamethasone acetate which are not encapsulated, and dexamethasone-encapsulated microspheres A-10, to evaluate the bioavailability improvement of semaglutide microspheres in Example 11.
Figs. 18 and 19 are experimental results on the minimum blood concentration causing side effects of drugs performed by using dexamethasone microspheres and dexamethasone acetate microspheres.

### [Mode of Invention]

Hereinafter, preferred embodiments are presented to aid understanding of the present invention, but the following examples are merely illustrative of the present invention, and various changes and modifications are possible within the scope and spirit of the present invention. It is obvious to those skilled in the art, it goes without saying that these variations and modifications fall within the scope of the appended claims.

### Example 1: Preparation of anti-inflammatory microspheres

### 1-1: Preparation of microspheres containing anti-inflammatory drugs

The disperse phase solution for preparing microspheres was prepared by mixing 1.2g of Resomer RG502H (iv = 0.16-0.24 dL/g; manufacturer: Evonik, Germany) as a biocompatible polymer in 6.00g of dichloromethane (manufacturer: JTBaker, USA) and by dissolving until it became transparent. Then, the solution was added with 0.8 g of dexamethasone (D50 = 1.8 µm; manufacturer: Farmabios, Italy), and used subsequently after being stirred for 5 minutes or more to sufficiently disperse.

The continuous phase solution for preparing microspheres was aqueous solution of 0.5% (w/v) polyvinyl alcohol (viscosity: 4.8-5.8 mPa s). 1,200 mL of the continuous phase solution was put into a preparation container and was added with the disperse phase solution at a flow rate of 6.0 mL per minute while stirring at a speed of 2,000 rpm with a high-speed stirrer (L4RT, Silverson, England), to form an emulsion. After the addition of disperse phase solution was completed, the emulsion was stirred at a speed of 200 rpm, while maintaining the temperature at 25 °C for 30 minutes, and then, heated to 45°C and maintained for 3 hours to evaporate and remove the organic solvent to create microspheres.

After the removal of the organic solvent was completed, the temperature of the microsphere suspension was lowered to 25 °C, and the residual polyvinyl alcohol was removed by filtration and washing with tertiary distilled water three times, to obtain microspheres. The microspheres obtained in this step were lyophilized to recover the final sustained-release microspheres containing dexamethasone. The anti-inflammatory microspheres prepared in this Example were named as microsphere A-1.

Additionally, anti-inflammatory microspheres A-2, A-3 and A-4 were prepared according to substantially the same method as anti-inflammatory microsphere A-1, except that the membrane emulsification method without using high-speed stirrer was performed differently under the conditions listed in Table 1 below.

Specifically, 3.5 g of Resomer R 203H (i.v. = 0.25-0.35 dL/g; manufacturer: Evonik, Germany) was used for preparing anti-inflammatory microsphere A-2. 3.5 g of Purasorb PDL 05 (i.v. = 0.4-0.6 dL/g; manufacturer: Purac, Netherlands) was used for preparing anti-inflammatory microsphere A-3. 0.78 g of Resomer RG 752H (i.v. = 0.14-0.22 dL/g; manufacturer: Evonik, Germany) and 1.82 g of Resomer RG 753H (i.v. = 0.32-0.44 dL/g; manufacturer: Evonik, Germany) were used for preparing anti-inflammatory microsphere A-4.

**[Table 1]**

| Category | A-1 | A-2 | A-3 | A-4 |
|---|---|---|---|---|
| polymer type | RG502H | R203H | PDL05 | RG752H:RG753H |
| Drug | Dexamethans one base | Dexamethans one base | Dexamethans one base | Dexamethansone base |
| Used amount of drug (g) | 0.8 | 1.5 | 1.5 | 1.4 |
| Used amount of polymer (g) | 1.2 | 3.5 | 3.5 | 2.6 |
| Used amount of disperse phase solvent (g) | 6 | 17.5 | 17.5 | 13 |
| Used amount of continuous phase solution (mL) | 1,200 | 3,500 | 3,500 | 2,600 |
| Drug content (w/w%) | 36.8 | 26 | 25.6 | 27.7 |
| Encapsulation efficiency of drug (%) | 92 | 86.7 | 85.3 | 79.1 |
| Microsphere size (D50) (µm) | 53.49 | 49.83 | 49.28 | 47.28 |

### 1-2: Preparation of microspheres containing anti-inflammatory drugs

The disperse phase solution for preparing microspheres was prepared by mixing 0.39g of RG 752H (iv 0.14-0.22 dl/g; manufacturer: Evonik, Germany), 0.91g of RG 753H (iv 0.32-0.44 dl/g; manufacturer: Evonik, Germany), and 0.70 g of dexamethasone acetate (manufacturer: Pfizer, USA) with 6.50 g of dichloromethane (manufacturer: JT Baker, USA) and 4.37 g of benzyl alcohol (manufacturer: Junsei, Japan).

The disperse phase solution was used after sufficiently dissolving by stirring for 30 minutes or more. The continuous phase solution was used by adding 2.5% (w/v) sodium chloride to an aqueous solution of 0.5% (w/v) polyvinyl alcohol (viscosity: 4.8-5.8 mPa s). 1,500mL of the continuous phase solution was connected to the emulsifier equipped with a porous membrane having a diameter of 40µm, and added with the prepared disperse phase solution at the same time, to prepare microsphere suspension. The microsphere suspension was put in a preparation container and stirred at a speed of 200 rpm, while maintaining the temperature of the preparation container at 25°C. After the addition of the disperse phase solution was completed, the organic solvent was removed while maintaining the temperature of the microsphere suspension at 45 °C for 3 hours. After the removal of the organic solvent was completed, the temperature of the microsphere suspension was lowered to 25 °C.

The dexamethasone acetate-containing microspheres had a drug content of 31.76% by weight based on 100% by weight of total content of polymer and drug, and 36.70 µm of the microsphere size (D50). The microspheres containing dexamethasone acetate prepared in this Example were named as microsphere A-5.

Additionally, anti-inflammatory microspheres A-6, A-7, A-8, A-9 and A-10 were prepared according to substantially the same method as anti-inflammatory microsphere A-5, except that the disperse phase solution was prepared and used under the conditions described in Table 2 below.

Specifically, for preparing anti-inflammatory microsphere A-6, the disperse phase solution was prepared by mixing 1.60 g of biocompatible polymer, Purasorb PDLG 5002A (i.v.= 0.16-0.24 dl/g; manufacturer: Purac, Netherlands) and 0.40 g of dexamethasone acetate (manufacturer: Pfizer, USA), with 4.00 g of dichloromethane (manufacturer: JT Baker, USA) and 2.50 g of benzyl alcohol (manufacturer: Junsei, Japan). For the preparation of anti-inflammatory microsphere A-7, the disperse phase solution was prepared by mixing 0.32 g Purasorb PDLG 7502A (i.v.= 0.16-0.24 dl/g; manufacturer: Purac, Netherlands) and 1.28 g of Purasorb PDL 04A (i.v.= 0.35-0.45 dl/g; manufacturer: Purac, The Netherlands) as biocompatible polymer, and 0.40 g of dexamethasone acetate (manufacturer: Pfizer, USA), with 4.00 g of dichloromethane (manufacturer: JT Baker, USA) and 2.50 g of benzyl alcohol (manufacturer: Junsei, Japan). For the preparation of anti-inflammatory microsphere A-8, the disperse phase solution was prepared by mixing 0.54 g of Purasorb PDL 02A (i.v.= 0.16-0.24 dl/g; manufacturer: Purac, Netherlands) and 1.26 g of RG 755S (i.v.= 0.50-0.70 dl/g; manufacturer: Evonik, Germany) as biocompatible polymers and 0.20 g of dexamethasone acetate (manufacturer: Pfizer, USA), with 4.50 g of dichloromethane (manufacturer: JT Baker, USA) and 1.25 g of benzyl alcohol (manufacturer: Junsei, Japan).

For the preparation of anti-inflammatory microsphere A-9, the disperse phase solution was prepared by mixing 1.40 g of R203H (i.v.= 0.25-0.35 dl/g; manufacturer: Evonik, Germany) as biocompatible polymer and 0.60 g of dexamethasone acetate (manufacturer: Pfizer, USA), with 3.50g of dichloromethane (Manufacturer: JT Baker, USA) and 3.74g of benzyl alcohol (manufacturer: Junsei, Japan).

For the preparation of anti-inflammatory microspheres A-10, the disperse phase solution was prepared by mixing 0.88 g Purasorb PDLG 7502A (i.v.= 0.16-0.24 dl/g; manufacturer: Purac, Netherlands), 0.18 g of RG 752H (i.v.= 0.14-0.22 dl/g; manufacturer: Evonik, Germany) and 0.41 g of RG 753H (iv 0.32-0.44 dl/g; manufacturer: Evonik, Germany) as biocompatible polymers and 0.54 g of dexamethasone acetate (manufacturer: Pfizer, USA), with 5.13g of dichloromethane (manufacturer: JT Baker , USA), 0.73g of dimethyl sulfoxide (manufacturer: JT Baker, USA) and 1.97g of benzyl alcohol (manufacturer: Junsei, Japan).

**[Table 2]**

| Category | A-5 | A-6 | A-7 |
|---|---|---|---|
| polymer type | RG752H:RG753H | PDLG 5002A | PDLG 7502A |
| | | | PDL 04A |
| Drug | Dexamethansone acetate | Dexamethansone acetate | Dexamethansone acetate |
| Used amount of drug (g) | 0.70 | 0.40 | 0.50 |
| Used amount of polymer(g) | 1.30 | 1.60 | 1.60 |
| Used amount of disperse phase solvent (g) | 10.87 | 6.50 | 6.50 |
| Used amount of continuous phase solution (mL) | 1,500 | 1,500 | 1,500 |
| Drug content (w/w%) | 31.76 | 19.66 | 22.09 |
| Encapsulation efficiency of drug (%) | 90.75 | 98.32 | 110.43 |
| Microsphere size (D50) (µm) | 36.70 | 40.27 | 40.19 |

**[Table 3]**

| Category | A-8 | A-9 | A-10 |
|---|---|---|---|
| polymer type | PDL-02A | R203H | PDLG 7502A |
| | RG 755S | | RG 752H |
| | | | RG 753H |
| Drug | Dexamethansone acetate | Dexamethansone acetate | Dexamethansone acetate |
| Used amount of drug (g) | 0.2 | 0.6 | 0.54 |
| Used amount of polymer(g) | 1.8 | 1.4 | 1.47 |
| Used amount of disperse phase solvent (g) | 5.75 | 7.24 | 6.83 |
| Used amount of continuous phase solution (mL) | 1,500 | 1,500 | 1,500 |
| Drug content (w/w%) | 10.9 | 31.96 | 25.21 |
| Encapsulation efficiency of drug (%) | 108.96 | 106.53 | 94.24 |
| Microsphere size (D50) (µm) | 42.76 | 39.03 | 48.12 |

### 1-3: Preparation of microspheres containing anti-inflammatory drugs

The disperse phase solution for preparing microspheres was prepared by mixing 0.45 g of RG 752H (i.v.= 0.14-0.22 dl/g; manufacturer: Evonik, Germany) and 0.45 g of RG 753H (i.v.= 0.32-0.44 dl/g; manufacturer: Evonik, Germany) as biocompatible polymers and 0.10 g of meloxicam (manufacturer: Swati, India), with 4.50 g of dichloromethane (manufacturer: JT Baker, USA) and 1.20 g of dimethyl sulfoxide (manufacturer: JT Baker, USA).

The disperse phase solution was used after sufficiently dissolving by stirring for 30 minutes or more. The continuous phase solution was used by adding 2.5% (w/v) sodium chloride to an aqueous solution of 0.5% (w/v) polyvinyl alcohol (viscosity: 4.8-5.8 mPa s). 2,000mL of the continuous phase solution was connected to the emulsifier equipped with a porous membrane having a diameter of 40µm, and added with the prepared disperse phase solution at the same time, to prepare microsphere suspension. The microsphere suspension was put in a preparation container and stirred at a speed of 200 rpm, while maintaining the temperature of the preparation container at 25°C. After the addition of the disperse phase solution was completed, the organic solvent was removed while maintaining the temperature of the microsphere suspension at 45 °C for 3 hours. After the removal of the organic solvent was completed, the temperature of the microsphere suspension was lowered to 25 °C.

The meloxicam-containing microspheres had a drug content of 9.82% by weight based on 100% by weight of the total content of polymer and drug, and 60.24 µm of microsphere size (D50). The microspheres containing meloxicam prepared in this Example were named as microsphere A-11.

### 1-4: Preparation of microspheres containing anti-inflammatory drugs

The disperse phase solution for preparing microspheres was prepared by mixing 0.40 g of RG 752H (i.v.= 0.14-0.22 dl/g; manufacturer: Evonik, Germany) and 0.40 g of RG 753H (i.v.= 0.32-0.44 dl/g; manufacturer: Evonik, Germany) as biocompatible polymer and 0.10 g of Ketorolac (manufacturer: Dr. Reddy's laboratories, India), with 4.00 g of dichloromethane (manufacturer: JT Baker, USA) and 0.60 g of dimethyl sulfoxide (manufacturer: JT Baker, USA). The preparation of the continuous phase solution and the microspheres were carried out in substantially the same method as the preparation method of the meloxicam-containing microspheres of Example 1-3 as described above, to prepare the ketorolac-containing microspheres.

The ketorolac-containing microspheres had a drug content of 12.30% by weight based on 100% by weight of the total content of polymer and drug, and 52.28 µm of microsphere size (D50). The microspheres containing ketorolac prepared in this Example were named as microspheres A-12.

### Example 2. Preparation of inflammatory microspheres

### 2-1: Manufacture of Placebo microspheres

The disperse phase solution for preparing microspheres was prepared by mixing 10 g of Resomer RG502H (i.v. = 0.16-0.24 dL/g; manufacturer: Evonik, Germany) as biocompatible polymer, with 90 g of dichloromethane (manufacturer: JTBaker, USA), and by dissolving until it became transparent.

The continuous phase solution was used by adding 2.5% (w/v) sodium chloride to an aqueous solution of 0.1% (w/v) polyvinyl alcohol (viscosity: 4.8-5.8 mPa s). 9,000 mL of the continuous phase solution was connected to the emulsifier equipped with a porous membrane having a diameter of 10µm, and added with the prepared disperse phase solution at the same time, to prepare microsphere suspension. The microsphere suspension was put in a preparation container and stirred at a speed of 200 rpm. The temperature of the membrane emulsifier and the preparation container were maintained at 25°C. After the addition of disperse phase solution was completed, the emulsion was stirred at a speed of 200 rpm with maintaining the temperature at 25 °C for 30 minutes, and then, heated to 45°C and maintained for 3 hours to evaporate and remove the organic solvent to create microspheres.

After the removal of the organic solvent was completed, the temperature of the microsphere suspension was lowered to 25 °C, and residual polyvinyl alcohol was removed by filtration and washing with tertiary distilled water three times, to obtain microspheres having an average diameter of 20 µm.

The disperse phase solution for preparing microspheres was prepared by mixing 30 g of Resomer RG502H (i.v. = 0.16-0.24 dL/g; manufacturer: Evonik, Germany) with 120 g of dichloromethane (manufacturer: JTBaker, USA), and by dissolving until it became transparent. The continuous phase solution was an aqueous solution of 0.5% (w/v) polyvinyl alcohol (viscosity: 4.8-5.8 mPa · s). 12,000 mL of the continuous phase was connected to the emulsifier equipped with a porous membrane having a diameter of 20µm, and added with the disperse phase solution at the same time, to obtain microsphere suspension. The preparation of microsphere emulsion, the evaporation and removal of organic solvent, and the washing were prepared in substantially the same manner as the microspheres having an average particle size of 20 µm above, to obtain microspheres with an average particle size of 30 µm.

The disperse phase solution for preparing microspheres was prepared by mixing 10 g of biocompatible polymer Resomer RG502H (i.v. = 0.16-0.24 dL/g; manufacturer: Evonik, Germany), with 18.57 g of dichloromethane (manufacturer: JTBaker, USA) and by dissolving until it became transparent. The continuous phase solution was an aqueous solution of 0.5% (w/v) polyvinyl alcohol (viscosity: 4.8-5.8 mPa s). 2,786 mL of the continuous phase was connected to the emulsifier equipped with a porous membrane having a diameter of 40µm, and was added with the disperse phase solution at the same time, to obtain microsphere suspension.. The preparation of microsphere emulsion, the evaporation and removal of organic solvent, and the washing were prepared in substantially the same manner as the microspheres having an average particle size of 20 µm, to obtain microspheres with an average particle size of 60 µm.

The biodegradable polymer microparticles having an average particle diameter of 20, 30, or 60 µm were prepared by the above method, and the inflammatory placebo microspheres prepared in this example were named as microspheres B-1.

The average particle diameter of the microparticles was analyzed using a laser diffraction particle size analyzer. 50 mg of the composition was mixed with 1 mL of ultrapure water with a vortex mixer for 20 seconds, and then dispersed by being placed in an ultrasonic generator for 1 minute. The dispersion was put into a particle size analyzer (Microtrac Bluewave, Japan) and measured for 20 seconds. The average particle diameter (Median Diameter) is expressed as D50 or D(v, 0.5) for a particle size corresponding to 50% by volume in the particle size distribution curve. As a result of particle size measurement, the measured D50 values of 20 µm, 30 µm, and 60 µm microspheres were 21.02 µm, 35.56 µm, and 56.49 µm, respectively.

### 2-2: Preparation of donepezil microspheres

The disperse phase solution used in the preparation of donepezil microspheres was prepared as follows. The disperse phase solution was prepared by mixing 580.1 g of biocompatible polymer of PLA (Resomer R 203H i.v.=0.25-0.35 dl/g; manufacturer Evonik, Germany / Resomer R 205S, i.v.= 0.55-0.75 dL/g; manufacturer: Evonik, Germany) and 396.1 g of donepezil base (manufacturer: Neuland Laboratories, India), with 3156.2 g of dichloromethane (manufacturer: JT Baker, USA). The disperse phase solution was used subsequently after sufficiently dissolving with stirring for 30 minutes or more.

The continuous phase solution was aqueous solution of 1% (w/v) polyvinyl alcohol (viscosity: 4.8-5.8 mPa s). The continuous phase solution was connected to the emulsifier equipped with a porous membrane having a diameter of 40µm, and added with the disperse phase solution at the same time, to obtain microsphere suspension. The microsphere suspension was put in a preparation container and stirred at a speed of 200 rpm, while maintaining the temperature of the preparation container at 25°C. After the addition of the disperse phase solution was completed, the organic solvent was removed whiling maintaining the microsphere suspension at 47.5°C for 4 hours. After the removal of the organic solvent was completed, the temperature of the microsphere suspension was lowered to 20 °C.

The donepezil-containing microspheres had a drug content of 31.3 wt% based on 100 wt% of the total content of polymer and drug, and 74.47 µm of microsphere size (D50). The donepezil microspheres prepared in this Example were named as microspheres B-2.

### 2-3: Preparation of rivastigmine microspheres

The disperse phase solution was prepared by mixing 2.00 g of Resomer RG 653H (i.v.=0.32-0.44 dL/g; manufacturer: Evonik, Germany) and 4.00 g of Resomer R 203H (i.v.=0.25-0.35 dL/g; manufacturer: Evonik, Germany) as biocompatible polymer and 2.70 g of rivastigmine (manufacturer: Hwail Pharmaceutical, Korea) and 2.09 g of pamoic acid (manufacturer: Amitychem, China), with 30.00 g of dichloromethane (manufacturer: JT Baker, USA) and by sufficiently dissolving until they became transparent. The biodegradable polymers were used as 653H:203H (1:2 of weight ratio).

The continuous phase solution was an aqueous solution of 0.5% (w/v) polyvinyl alcohol (viscosity: 4.8 to 5.8 mPa · s). 4,500 mL of the continuous phase was connected to the emulsifier equipped with a porous membrane having a diameter of 30µm, and added with the disperse phase solution at the same time, to obtain microsphere suspension.. The microsphere suspension was stirred at a speed of 200 rpm in a preparation container. The temperature of the membrane emulsifier and the preparation container were maintained at 25 °C, and after the addition of the disperse phase solution was completed, the organic solvent was removed while maintaining the temperature of microsphere suspension at 45 ° C for 3 hours. After the removal of the organic solvent was completed, the temperature of the microsphere suspension was lowered to 25 °C. The microsphere suspension was repeatedly washed several times with ultrapure water to remove residual polyvinyl alcohol, and the microspheres were lyophilized.

The rivastigmine microspheres had a drug content of 21.24% by weight based on 100% by weight of the total content of polymer and drug, and 49.02 µm of microsphere size (D50). The rivastigmine microspheres prepared in this Example were named as microspheres B-3.

### 2-4: Preparation of finasteride microspheres

The disperse phase solution was prepared by mixing 0.60g of biocompatible polymer Purasorb PDL 02A (i.v.=0.16-0.24 dL/g; manufacturer: Purac, Netherlands) and 0.40g of finasteride (manufacturer: Aurobindo pharma, India), with 5.40 g of dichloromethane (manufacturer: JT Baker, USA) and by sufficiently dissolving until it became transparent.

The continuous phase solution was an aqueous solution of 0.5% (w/v) polyvinyl alcohol (viscosity: 4.8 to 5.8 mPa · s). 1,500 mL of the continuous phase was connected to the emulsifier equipped with a porous membrane having a diameter of 20µm, and added with the disperse phase solution at the same time, to obtain microsphere suspension. The microsphere suspension was stirred at a speed of 200 rpm in a preparation container. The temperature of the membrane emulsifier and the preparation container were maintained at 25 °C, and after the addition of the disperse phase solution was completed, the organic solvent was removed while maintaining the microsphere suspension temperature at 40 °C for 3 hours. After the removal of the organic solvent was completed, the temperature of the microsphere suspension was lowered to 25 °C. The microsphere suspension was repeatedly washed several times with ultrapure water to remove residual polyvinyl alcohol, and the microspheres were lyophilized.

The finasteride microspheres had a drug content of 42.6% by weight based on 100% by weight of the total content of polymer and drug, 106.5% of the drug encapsulation efficiency and 40.43 µm of the microsphere size (D50). The finasteride microspheres prepared in this Example were named as microspheres B-4.

### 2-5: Preparation of semaglutide microspheres

The disperse phase solution was prepared by mixing 0.9 g of Purasorb PDLG 7504A (i.v.= 0.38-0.48 dl/g, Purac, The Netherlands) as a biocompatible polymer and 0.1 g of semaglutide (manufacturer: Chengdu, China) as a drug, with 17.14 g of dichloromethane (manufacturer: JT Baker, USA) and 3.42 g of glacial acetic acid (manufacturer: Daejeong, Korea), and by sufficiently dissolving until it became transparent.

The continuous phase solution was an aqueous solution of 0.1% (w/v) polyvinyl alcohol (viscosity: 4.8 to 5.8 mPa s). 2.0 L of the continuous phase solution was connected to the emulsifier equipped with a porous membrane having a diameter of 40µm, and added with the disperse phase solution at the same time, to obtain microsphere suspension. The microsphere suspension was stirred at a speed of 300 rpm in a preparation container. The temperature of the membrane emulsifier and the preparation container were maintained at 25 °C, and after the injection of the disperse phase, the organic solvent was removed while maintaining the microsphere suspension temperature at 40 °C for 3 hours. After the removal of the organic solvent, the temperature of the microsphere suspension was lowered to 25 °C. The microsphere suspension was repeatedly washed several times with ultrapure water to remove residual polyvinyl alcohol, and the microspheres were lyophilized.

The semaglutide-containing microspheres had a drug content of 9.10 wt% based on 100 wt% of the total content of polymer and drug, and 41.77 µm of the microsphere size (D50). The semaglutide microspheres prepared in this Example were named as microspheres B-5.

### 2-6: Preparation of semaglutide microspheres (GB-7001-717 formulation, additional formulation))

The disperse phase solution was prepared by mixing 1.85 g of Resomer RG 503H (I.V.=0.32-0.44 dL/g; manufacturer: Evonik, Germany) and 3.70 g of Resomer RG 653H (I.V.=0.32-0.44 dL/g; manufacturer: Evonik, Germany) as biocompatible polymers and 0.45 g of semaglutide (manufacturer: Chengdu, China) as a drug, with 48.6 g of dichloromethane (manufacturer: JT Baker, USA) and 25.2 g of glacial acetic acid (manufacturer: Daejeong, Korea) and by dissolving sufficiently until it became transparent.

The preparation of the continuous phase solution and the microspheres were carried out in substantially the same manner as the preparation method of the semaglutide-containing microspheres of Example 2-5, to prepare and lyophilize the semaglutide-containing microspheres.

The semaglutide-containing microspheres had a drug content of 6.92 wt% based on 100 wt% of the total content of polymer and drug, and 36.20 µm of the microsphere size (D50). The semaglutide microspheres prepared in this Example were named as microspheres B-6.

### 2-7: Preparation of octreotide microspheres

The disperse phase solution was prepared by mixing 0.65 g of Purasorb PDLG 7504A (i.v.= 0.38-0.48 dl/g, Purac, The Netherlands) as biocompatible polymer and 0.30 g of octreotide acetate (manufacturer: Zhejiang Peptites Biotech Co., Ltd, China) and 0.05 g of pamoic acid (manufacturer: of Amitychem, China or Kyeongbo Pharmaceutical, Korea), with 5.80 g of dichloromethane (manufacturer: JT Baker, USA) and 1.90 g of dimethyl sulfoxide (manufacturer: Samchun Chemical, Korea) and by dissolving sufficiently until it became transparent to the naked eye.

The continuous phase solution was prepared by adding 2.5% (w/v) sodium chloride to an aqueous solution of 0.1% (w/v) polyvinyl alcohol (viscosity: 4.8-5.8 mPa s). 2 L of the continuous phase solution was connected to the emulsifier equipped with a porous membrane having a diameter of 10µm, and added with the disperse phase solution at the same time, to obtain microsphere suspension. The microsphere suspension was stirred at a speed of 200 rpm in a preparation container. The temperature of the membrane emulsifier and the preparation container were maintained at 25 °C, and after the addition of the disperse phase was completed, the organic solvent was removed while maintaining the microsphere suspension temperature at 40 °C for 3 hours. After the removal of the organic solvent was completed, the temperature of the microsphere suspension was lowered to 25 °C. The microsphere suspension was repeatedly washed several times with ultrapure water to remove residual polyvinyl alcohol, and the microsphere was lyophilized.

The octreotide-containing microspheres had a drug content of 21.75 wt% based on 100 wt% of the total content of polymer and drug, and 30.59 µm of the microsphere size (D50). The microspheres prepared in this example were named as microspheres B-7.

### 2-8. Preparation of leuprolide microspheres

The disperse phase solution was prepared by mixing 3.6 g of Purasorb PDLG 7502A (i.v.= 0.16-0.24 dl/g, Purac, The Netherlands) as biocompatible polymer and 0.444 g of leuprolide acetate (manufacturer: Polypeptide Laboratories Pvt, Ltd., India) with 6.0g of dichloromethane (manufacturer: JT Baker, USA) and 1.722 g of methyl alcohol (manufacturer: Tedia Company, USA) and by dissolving sufficiently until it became transparent to the naked eye.

The continuous phase solution was an aqueous solution of 1.0% (w/v) polyvinyl alcohol (viscosity: 4.8-5.8 mPa s). 2.5 L of the continuous phase solution was connected to the emulsifier equipped with a porous membrane having a diameter of 20µm, and added with the disperse phase solution at the same time, to obtain microsphere suspension. The microsphere suspension was stirred at a speed of 200 rpm in a preparation container. The temperature of the membrane emulsifier and the container, and the microsphere suspension were maintained at 15 °C. After the addition of the disperse phase solution was completed, the microsphere suspension was stirred for 3 hours. Then, 5L of continuous phase solution of 0.5% (w / v) polyvinyl alcohol added with 10% ethyl alcohol (manufacturer: Samchun Chemical, Korea) was exchanged twice to remove the organic solvent. After the removal of the organic solvent was completed, the microsphere suspension was repeatedly washed several times with ultrapure water to remove residual polyvinyl alcohol, and the microsphere was lyophilized.

The microspheres containing leuprolide had a drug content of 10.07% by weight based on 100% by weight of the total content of polymer and drug, and 32.42 µm of the microsphere size (D50). Microspheres prepared in this example were named as microspheres B-8.

### 2-9: Preparation of microspheres containing leuprolide and dexamethasone acetate

The disperse phase solution was prepared by mixing 3.594 g of Purasorb PDLG 7502A (i.v.= 0.16-0.24 dl/g, Purac, The Netherlands) as biocompatible polymer, 0.448 g of leuprolide acetate (manufacturer: Polypeptide Laboratories Pvt, Ltd., India) and 0.0064 g of dexamethasone acetate, with 6.0 g of dichloromethane (manufacturer: JT Baker, USA) and 1.739 g of methyl alcohol (manufacturer: Tedia Company, USA), and by dissolving sufficiently until it became transparent to the naked eye.

Preparation of the continuous phase and the microspheres were carried out in substantially the same manner as the method for preparing the microspheres containing leuprolide of Example 2-8, and the microspheres containing leuprolide and dexamethasone acetate were prepared and lyophilized. The drug microspheres prepared in this Example were named as microspheres B-9.

### 2-10: Preparation of microspheres containing leuprolide and dexamethasone base

The disperse phase solution was prepared by mixing 3.587 g of Purasorb PDLG 7502A (i.v. 0.16-0.24 dl/g, Purac, The Netherlands) as a biocompatible polymer, 0.448 g of leuprolide acetate (manufacturer: Polypeptide Laboratories Pvt, Ltd., India) and 0.0128 g of dexamethasone base, with 6.0 g of dichloromethane (manufacturer: JT Baker, USA) and 1.739 g of methyl alcohol (manufacturer: Tedia Company, USA) and by dissolving sufficiently until it became transparent to the naked eye.

The preparation of the continuous phase and the microspheres were carried out in substantially the same manner as the preparation method of the microspheres containing leuprolide of Example 2-8, and the microspheres containing leuprolide and dexamethasone free base were prepared and lyophilized. The drug microspheres prepared in this Example were named as microspheres B-10.

The properties of the microspheres prepared in Examples 2-8, 2-9, and 2-10 were represented in Table 4

**[Table 4]**

| Category | B-8 | B-9 | B-10 |
|---|---|---|---|
| Used amount of drug (a) | 0.444 | 0.448 | 0.448 |
| Used amount of dexamethasone (g) | 0 | 0.0064 (Dexamethasone Acetate) | 0.0128 (dexamethasone base) |
| Used amount of polymer (g) | 3.6 | 3.594 | 3.587 |
| Drug content (%) | 10.07 | 9.88 | 8.36 |
| Encapsulation efficiency of drug (%) | 100.7 | 98.8 | 83.6 |
| Dexamethasone content (%) | 0 | 0.15 (dexamethasone acetate) | 0.16 (dexamethasone base) |
| Encapsulation efficiency of dexamethasone (%) | 0 | 95.1 (dexamethasone acetate) | 49.6 (dexamethasone base) |
| Microsphere size (D50) | 32.42 | 34.88 | 35.02 |

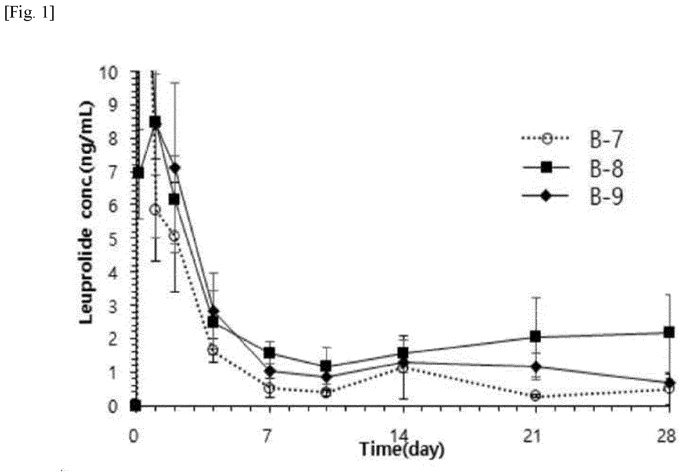

The bioavailability improvement effects of the microspheres prepared in Examples 2-8, 2-9, and 2-10 are shown in Table 5 and FIG. 1.

**[Table 5]**

| Category | B-8 | B-9 | B-10 |
|---|---|---|---|
| Dose (mg/head) | 1.5 | 1.5 | 1.5 |
| AUC₀₋₂₈ (ng*day/mL) | 40.4 | 59.5 | 67.4 |
| Cmax (ng/mL) | 57.8 | 60.4 | 44.4 |
| Tmax(day) | 0.04 | 0.04 | 0.04 |

As shown in Table 5, based on 100 of the bioavailability (AUC₀₋₂₈) of dexamethasone-free leuprolide microspheres (AUC ₀₋₂₈), the bioavailability of leuprolide in the microspheres containing leuprolide and dexamethasone was 147% for the combined use of dexamethasone acetate, and was improved to 167% for the combined use of dexamethasone free base.

### 2-11: Preparation of deslorelin microspheres

The disperse phase solution was prepared by mixing 8.57g of Purasorb PDLG 7502A (i.v.= 0.16-0.24 dl/g; manufacturer: Purac, Netherlands) and 31.43 g of Purasorb PDL 04A (i.v.= 0.35-0.45 dl/g; manufacturer Purac, Netherlands) at a weight ratio of 21:79 as biocompatible polymers and 10.00 g of deslorelin acetate (manufacturer: Chengdu, China), with 150.20g of dichloromethane (manufacturer: JT Baker, USA) and 93.96g of N-methyl-2-pyrrolidone (manufacturer: Ashland, India), and was used after being dissolved sufficiently with stirring for 30 minutes or more.

The continuous phase solution was an aqueous solution of 2.0% (w/v) polyvinyl alcohol (viscosity: 4.8 to 5.8 mPa · s). While operating a high-speed stirrer (Verso UHS, Silverson, England) sequentially at 1,700 and 1,400 rpm, the continuous phase solution and the disperse phase solution were put into an emulsifier together at the same time, to obtain microsphere suspension. The microsphere suspension was put in a preparation container and stirred at a speed of 200 rpm, and the temperature of the preparation container was maintained at 25°C. After the addition of disperse phase was completed, the organic solvent was removed while maintaining the temperature of the microsphere suspension at 45 °C for 3 hours. After the removal of the organic solvent was completed, the temperature of the microsphere suspension was lowered to 25°C, and the microsphere suspension was repeatedly washed with ultrapure water several times to remove residual polyvinyl alcohol, and the microspheres was lyophilized.

The deslorelin-containing microspheres had 13.52% by weight of drug content based on 100% by weight of the total content of polymer and drug, and 50.84 µm of the microsphere size (D50). The microspheres containing deslorelin prepared in this Example were named as microspheres B-11.

### 2-12: Preparation of microspheres containing deslorelin and dexamethasone base

The disperse phase solution was prepared by mixing 0.93 g of Purasorb PDLG 7502A (i.v. 0.16-0.24 dl/g; manufacturer: Purac, Netherlands) and 3.41 g of Purasorb PDL 04A (i.v. 0.35-0.45 dl/g; manufacturer Purac, Netherlands) at a weight ratio of 21:79 as biocompatible polymers, and 1.04 g of deslorelin acetate (manufacturer: Chengdu, China) and 0.023 g of dexamethasone base (manufacturer: Pfizer, USA), with 16.29 g of dichloromethane (manufacturer: JT Baker, USA) and 10.19 g of N-methyl-2-pyrrolidone (manufacturer: Ashland, India).

The preparation of the continuous phase and the microspheres were carried out in substantially the same manner as the method for preparing the microspheres containing deslorelin of Example 2-11, and the microspheres containing deslorelin and dexamethasone base were prepared and lyophilized.

The microspheres containing the base of deslorelin and dexamethasone had 13.25 % by weight of deslorelin content and 0.16 % by weight of dexamethasone base content, based on 100 % by weight of the total content of polymer and drug, and 43.28 µm of microsphere size (D50). The microspheres containing deslorelin and dexamethasone base prepared in this example were named as microspheres B-12.

### 2-13: Preparation of entecavir microspheres

The disperse phase solution for the preparation of entecavir microspheres was prepared by dissolving 0.53g of Resomer RG858S (i.v. = 1.3-1.7 dL/g; manufacturer: Evonik, Germany) and 0.23g of 502H (i.v. = 0.16-0.24 dL/g; manufacturer: Evonik, Germany) at 70:30 of weight ratio as biocompatible polymers, and 0.25g of entecavir (manufacturer: Kyongkyung Pharmaceutical, Korea), with 3.60g of dichloromethane (manufacturer: JTBaker, USA) and 5.39 g of dimethyl sulfoxide and then mixed and used after stirring sufficiently to make it transparent to the naked eye.

The continuous phase solution for the preparation of microspheres was used by adding 2.5 wt% of NaCl to an aqueous solution of 0.5% (w/v) polyvinyl alcohol (viscosity: 4.8-5.8 mPa s). 800 mL of the continuous phase solution was put into a preparation container and stirred at a speed of 3000 rpm with a high-speed stirrer (L4RT, Silverson, England), while adding the disperse phase solution at a flow rate of 10.0 mL per minute to form an emulsion. After the addition of disperse phase was completed, the organic solvent was removed by heating the emulsion to 45 °C and maintaining the temperature for 2 hours, while stirring at a speed of 200 rpm. Thereafter, the temperature of the microsphere suspension was cooled to 25 °C, and the residual polyvinyl alcohol was removed by filtration and repetitive washing with tertiary distilled water, to obtain microspheres. The obtained microspheres were lyophilized to recover sustained release microspheres containing entecavir.

The entecavir-containing microspheres had a drug content of 22.50% by weight based on 100% by weight of the total content of polymer and drug, and 63.63 µm of the microsphere size (D50). The anti-inflammatory microspheres prepared in this example were named as microspheres B-13.

### Example 3: Evaluation for the inflammatory properties of microspheres

Whether or not inflammation was induced in animals by administering microspheres was tested using rats. Drug-free placebo microspheres having an average particle size of 20 µm and 60 µm prepared in Example 2-1 were used as an inflammatory substance.

Animal experiments were performed according to the Animal Experimentation Ethics Committee regulations. Specifically, after purchasing male 5-week-old SD (Sprague-Dawley) rats and acclimatizing them for one week, they were divided into two groups of four rats per group. The placebo microspheres having an average particle diameter of 20 µm or 60 µm prepared in Example 2-1 were subcutaneously injected at a dose of 120 mg/head, respectively, under the skin of the dorsal side of rat. On 3rd and 8th days after administration, two rats per group were sacrificed, and the injection region was excised to evaluate the difference in the degree of inflammation induction, depending on the particle size of the injected microspheres in the acute and chronic inflammatory stages.

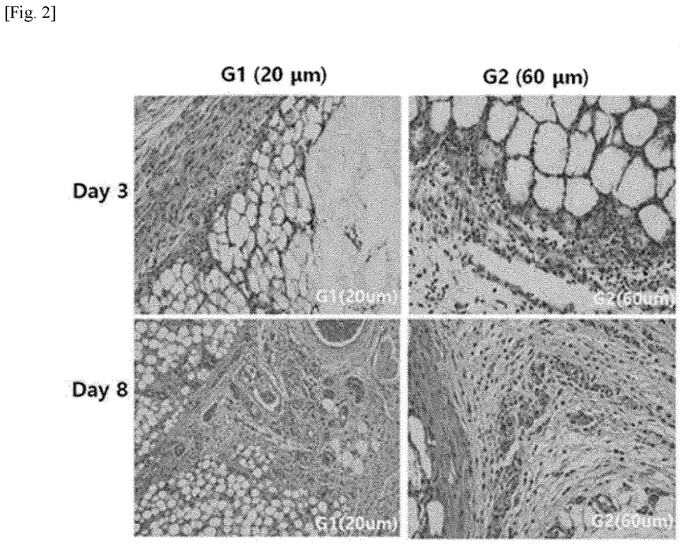

In order to measure the degree of inflammation, the excised administration region was fixed and embedded in neutral formalin, to make a paraffin block. The paraffin blocks were cut into 4 to 5 µm thick slices and stained with H&E (hematoxylin & eosin). Then, they were examined for the degree of inflammatory cell infiltration, angiogenesis and fibrous tissue formation using an optical microscope, to show the resulting photograph in FIG. 2.

In Table 6, the degree of inflammatory cell infiltration was determined by randomly selecting five fields in a 400-fold magnification field of view using an optical microscope on stained histopathology slides to evaluate the inflammatory cell infiltration, to represent them as "grade 0" for ≤ 5(or lower) of number, "grade 1" for 6 to 20, "grade 2" for 21 to 50, and "grade 3" for over 50. In Table 6, they were represented as the average value of each group. In addition, the formation of fibrous connective tissue was represented as the total number of animals which showed the formation of fibrous connective tissue, in each group.

**[Table 6]**

| Category | G3-1 | G3-2 |
|---|---|---|
| Inflammatory cell infiltration (Day 3) | 3 | 3 |
| Inflammatory cell infiltration (Day 8) | 2.5 | 1.5 |
| Fibrous tissue formation (Day 3) | 0.5 | 0 |
| Fibrous tissue formation (Day 8) | 2 | 2 |

As shown in the photograph of FIG. 2, the angiogenesis was not observed at the site of administration on the 3rd day after administration, which was the acute inflammation stage, and the site of administration on the 8th day of chronic inflammation stage, in both groups administered with 20 µm and 60 µm size placebo microspheres.

At 3rd day after administration which was the acute inflammation stage, the infiltration of inflammatory cells was in both groups administered with 20 µm and 60 µm size placebo microspheres, and weak fibrous tissue formation were in group administered with 20 µm size placebo microspheres. At 8th day of chronic inflammation stage, similar infiltration of inflammatory cells and formation of fibrous tissue were observed for at the administration sites of all animals of the groups administered with 20 µm and 60 µm placebo microspheres. Considering the above results, the inflammation induction caused by microsphere injection was observed, when 20µm-sized placebo microspheres and 60µm-sized placebo microspheres were administered subcutaneously once.

### Example 4: Efficacy test for combined administration of anti-inflammatory microspheres

0.94 mg (0.35 mg based on active ingredient content) or 0.23 mg (0.087 mg based on active ingredient content) of dexamethasone microspheres (A-1) in which 36.8% by weight of dexamethasone was encapsulated as dexamethasone free base from prepared in Example 1-1, were mixed respectively with 120 mg of 20 µm-sized placebo microspheres, or 120 mg of 60 µm-sized placebo microspheres prepared in Example 2-1, to obtain mixed formulations.

In substantially the same manner as in Example 3, the mixed formulations were subcutaneously administered to the dorsal side of SD rats, and two animals for each experimental group were sacrificed at 3^{rd} day and 8th day after administration, respectively. The administration site of the sacrificed rat was excised and prepared to histopathology sample slides, to test the anti-inflammatory effects according to the administration of dexamethasone, or administered amount of dexamethasone in acute inflammatory phase and chronic inflammatory phase.

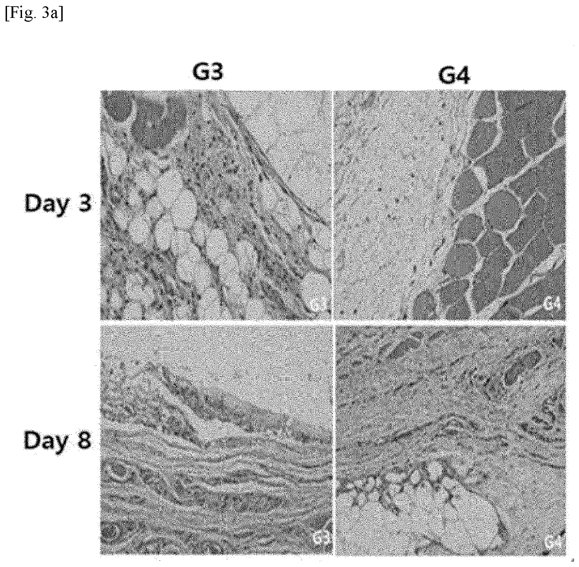

The experimental results are shown in FIG. 3a and FIFG. 3b, and Table 7, the degree of inflammatory cell infiltration and the formation of fibrous tissue at the administration site were evaluated in substantially the same manner as in Example 2.

**[Table 7]**

| Experimental group | G4-1 | G4-2 | G4-3 | G4-4 |
|---|---|---|---|---|
| Dosage of inflammatory microspheres (B-1) (mg/head) | 20µm Placebo (120 mg/head) | 60µm Placebo (120mg/head) | 20µm Placebo (120mg/head) | 60µm Placebo (120mg/head) |
| Dosage of dexamethasone microspheres (A-1) (based on active ingredient content) | 0.35 mg/head | 0.35 mg/head | 0.087 mg/head | 0.087 mg/head |
| Average inflammatory cell infiltration (day 3) | 0.5 | 0.5 | 2 | 1 |
| Average inflammatory cell infiltration (day 8) | 0 | 0.5 | 1.5 | 1 |
| Average fibrous tissue formation (day 3) | 0 | 0 | 0 | 0 |
| Average fibrous tissue formation (day 8) | 0 | 0 | 0 | 0.5 |

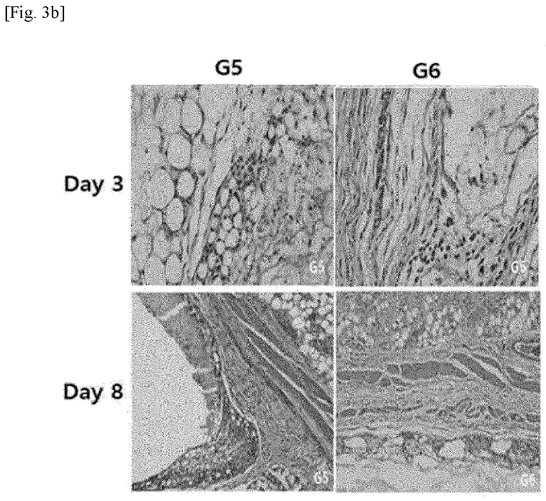

As shown in the pictures of FIGS. 3a and 3b, the angiogenesis was not observed at each administration site on the 3rd day after administration, which is the acute inflammation-inducing stage, and on the 8th day, which is the chronic inflammation stage, for all experimental groups subcutaneously injected with the placebo microspheres having an average particle diameter of 20 µm or 60 µm prepared in Example 2-1 and the anti-inflammatory dexamethasone microspheres prepared in Example 1-1.

As a result of the experiments, in all experimental groups G4-1 to G4-4 administered with a mixed formulation of placebo microspheres and dexamethasone microspheres, the inflammatory cell infiltration was decreased than those of G3-1 and G3-2 administered only with placebo microspheres at the site of administration on the stage of acute inflammation of day 3 after administration. In experimental groups G4-1 and G4-2 administered with a mixed formulation containing dexamethasone microspheres at high dose of 0.94mg/head (0.35mg/head as dexamethasone free base), the reduction of the inflammatory cell infiltration was even greater than those of experimental groups G4-3 and G4-4 administered with a mixed formulation containing dexamethasone microspheres at low dose of 0.87mg/head (0.23mg/head as dexamethasone free base). On the stage of acute inflammation of day 3 after administration, the fibrous tissue formation was not observed in all experimental groups.

As a result of the above experiment, in the experimental groups G4-1 to G4-4 administered with the mixed formulation of placebo microspheres and dexamethasone microspheres, the inflammatory cell infiltration was not observed for G4-1 at the administration site on the 8th day after administration, which is the chronic inflammation-inducing stage. However, the inflammatory cell infiltration was observed for G4-2 to G4-4, but was less than those of G3-1 to G3-2 administered with only placebo microspheres. On the 8th day after administration, which is the chronic inflammation-inducing stage, fibrous tissue formation with an average grade of 0.5 was observed only in G4-4 group.

Through the above results, it was confirmed that when dexamethasone microspheres were administered in combination, the degree of inflammation generation was suppressed compared to when placebo microspheres used as inflammation-inducing microspheres were administered only.

### Example 5. Drug release profile characteristics of anti-inflammatory microspheres

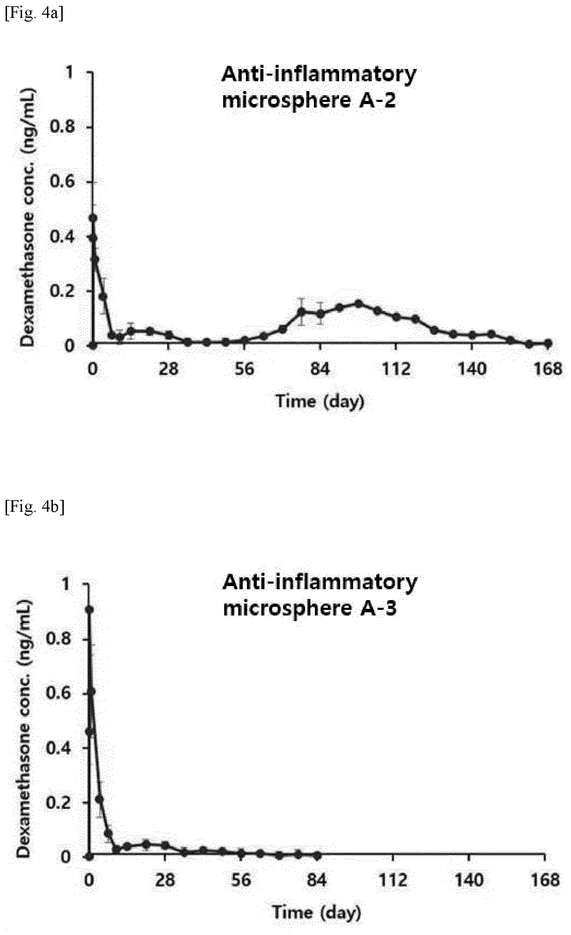

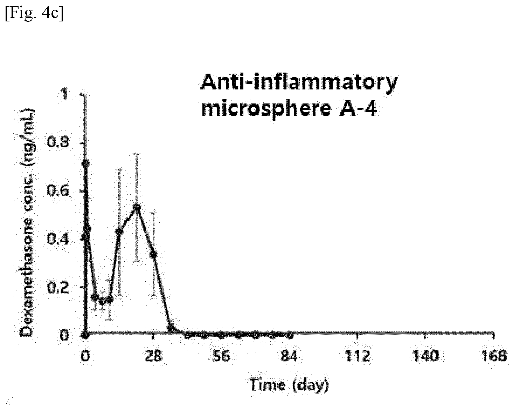

Pharmacokinetics of the anti-inflammatory microspheres A-2, A-3, and A-4 of Example 1 were evaluated using 9-week-old SD (Sprague-Dawley) rats. The anti-inflammatory microspheres A-2, A-3, and A-4 injection formulations of Example 1 were measured in rats at 0.06 mg/head, suspended in 0.3 mL of a dispersing solvent, and subcutaneously injected into SD rats. 0.25 to 0.5 mL of blood was collected for rats at pre-scheduled times, and the blood concentration of dexamethasone was measured using LC-MS/MS. The measurement results are shown in FIGS. 4A to 4C.s

As shown in Figs. 4A to 4C, it was confirmed that dexamethasone was released up to 168 days for anti-inflammatory microspheres A-2, up to 84 days for anti-inflammatory microspheres A-3, and up to 42 days for anti-inflammatory microspheres A-4. The Cmax of anti-inflammatory microspheres A-2 was 0.5 ng/mL, the Cmax of anti-inflammatory microspheres A-3 was 0.9 ng/mL, and the Cmax of anti-inflammatory microspheres A-4 was 0.7 ng/mL, all of which were less than 1 ng/mL. In particular, it was confirmed that anti-inflammatory microspheres A-2 and anti-inflammatory microspheres A-3 drew a graph with smooth release pattern. From the above results, it was confirmed that all of the microspheres A-2, A-3 and A-4 were formulations releasing dexamethasone slowly.

### Example 6. Efficacy test for anti-inflammatory microspheres

30 µm-sized placebo microspheres (B-1) prepared in Example 2-1 were used as microspheres for inducing inflammation. In substantially the same manner as in Example 3, the microspheres for inducing inflammation alone (G6-1) and mixed formulations of the inflammatory microspheres and dexamethasone microspheres (G6-2 to G6-5) were prepared, and microspheres for inducing inflammation were subcutaneously at the dorsal side of SD rats at a dose of 120 mg/head or 240 mg/head.

In order to test whether the anti-inflammatory effect of dexamethasone is maintained until almost all of the administered placebo microspheres are biodegraded, the time point of the 33rd day after administration of placebo microspheres was added, besides the 3rd and 8th days after administration, which is the time point of confirming inflammation in Example 3.

Dexamethasone microspheres were administered in a mixed formulation with the inflammatory microspheres at a dose of 0.22 mg/head (0.08 mg/head based on active ingredient content), 0.82 mg/head (0.3 mg/head based on active ingredient content) or 1.63 mg/head (0.6 mg/head based on active ingredient content). The specific experimental groups were G6-1 to G6-5, and are shown in Table 8 below. A total of 13 animals were included in each experimental group, and 5 animals on the 3rd day, 4 animals on the 8th day, and 4 animals on the 33rd day were allocated in each group.

In the table below, the dosage of inflammatory microspheres is represented as mg/head based on microspheres, and the dosage of anti-inflammatory microspheres is represented as mg/head based on the content of dexamethasone as an active ingredient.

**[Table 8]**

| Experim ental group | Dose of inflammatory microspheres (B-1) (mg/head) | Dose of active ingredient injected in anti-inflammatory microspheres |
|---|---|---|
| G6-1 | 120 mg/head | Untreated |
| G6-2 | 120 mg/head | 0.08 mg/head |
| G6-3 | 120 mg/head | 0.3 mg/head |
| G6-4 | 120 mg/head | 0.6 mg/head |
| G6-5 | 240 mg/head | 0.6 mg/head |

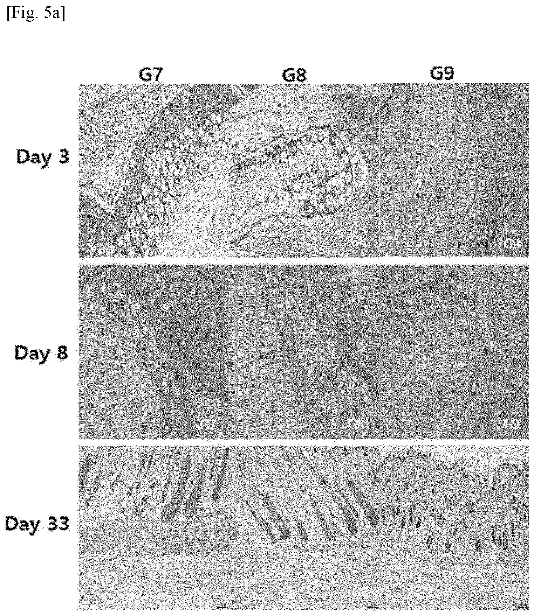

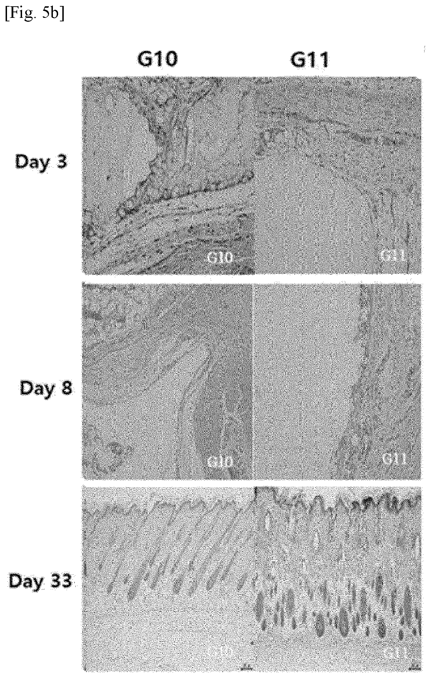

At each time point, the histopathological slide specimens were prepared using the same method as in Example 3, and inflammatory cell infiltration, angiogenesis, and fibrous tissue formation were observed. In addition, collagen deposition at the administration site was analyzed through collagen staining of histopathological slide specimens. Photographs of the stained specimens are shown in Figs. 5A and 5B.

The degree of inflammatory cell infiltration and the fibrous tissue formation at the administration site were evaluated in substantially the same manner as in Example 3, and are shown in Table 9 below.

**[Table 9]**

| Category | | G6-1 | G6-2 | G6-3 | G6-4 | G6-5 |
|---|---|---|---|---|---|---|
| inflammatory cell infiltration | Day3 | 3 | 1.6 | 1.4 | 0.6 | 0.8 |
| | Day 8 | 3 | One | 0.25 | 0 | 0.5 |
| | Day33 | 0 | 0 | 0 | 0 | 0 |
| angiogenesis | Day3 | 0 | 0 | 0 | 0 | 0 |
| | Day 8 | 4 | 0 | One | One | One |
| | Day33 | 0 | 0 | 0 | 0 | 0 |
| formation of fibrous connective tissue | Day3 | 0 | 0 | 0 | 0 | 0 |
| | Day 8 | 4 | 0 | 0 | 0 | 0 |
| | Day33 | 0 | 0 | 0 | 0 | 0 |

As shown in the analysis results of Table 9, in the case of the G6-1 group administered with only 120 mg/head of 30 µm placebo, an average grade 3 of inflammatory cell infiltration was observed on the 3rd day after administration when acute inflammation occurred, and an average grade 3 of inflammatory cell infiltration, formation of fibrous tissue and angiogenesis were observed for all animals on the 8th day after administration when chronic inflammation occurred inflammatory cell infiltration.

G6-2 to G6-5 administered with a mixed preparation of dexamethasone microspheres and 30µm placebo microspheres (B-1) of inflammatory microspheres, showed lower inflammatory cell infiltration in all animals than those of G6-1 administered with only placebo microspheres on the 3rd day after administration when acute inflammation occurred, represented lower grade of inflammatory cell infiltration as the dose of dexamethasone as an active ingredient increased, and did not showed angiogenesis and fibrous connective tissue formation.

On the 8th day after administration, when chronic inflammation occurred, average grade 3 of inflammatory cell infiltration, angiogenesis and the fibrous tissue formation were observed in all animals in G6-1 without administration of dexamethasone microspheres, but low grade of inflammatory cell infiltration was observed in G6-2, G6-3, and G6-5 administered with the mixed formulation, and the inflammatory cell infiltration was not observed in G6-4.

In G6-5 group administered with 240mg/head of 30µm placebo microspheres and 1.63mg/head of dexamethasone microspheres (0.6mg/head based on active ingredient content), average grade 0.8 of inflammatory cell infiltration was observed on the 3rd day after administration when acute inflammation occurred, and average grade 0.5 of inflammatory cell infiltration was observed at injection site on the 8th day after administration when chronic inflammation occurred, and the angiogenesis was observed in one animal. All inflammation-related symptoms observed in all treatment animals recovered on day 33.

According to the results, it was confirmed that the combined administration of inflammatory microspheres and dexamethasone microspheres suppressed the infiltration of inflammatory cells and the angiogenesis in the acute and chronic inflammatory stages compared to the group administered only with inflammatory microspheres. In particular, at the chronic inflammatory stage, the formation of fibrous connective tissue was completely suppressed in the group administered with dexamethasone microspheres.

### Example 7. Anti-inflammation test for combined administration of drug microspheres and anti-inflammatory microspheres

### 7-1: Combined use of leuprolide microspheres and anti-inflammatory microspheres

After evaluating that the inflammation induced by administering the drug-free placebo microspheres was alleviated by being co-administered with dexamethasone microspheres, this test was performed to investigate the anti-inflammatory effect of dexamethasone microspheres on the inflammation induced by the drug-containing microspheres.

As leuprolide microspheres, Leuplin which is a commercially available leuprolide microsphere that maintains its medicinal effect for 3 months, was purchased and used in this experiment with naming as microsphere B-14. 9-week-old male SD (Sprague-Dawley) rats were assigned to 3 groups of 9 rats each, and then was injected by Leuplin once subcutaneously into the dorsal side of the rats at a dose of 0.5 mL/head. This experiment is indicated as experimental group G7-1 in Table 10 below.

In addition to 2.7 mg/head (based on the content of the leuprolide free base) of the inflammation-inducing leuprolide microspheres, 0.23 mg/head of dexamethasone microspheres (0.06 mg/head based on active ingredient content) or 1.04 mg/head of dexamethasone microspheres (0.27 mg/head based on active ingredient content) was added to prepare mixed formulations (G7-2 to G7-3), and was administered once subcutaneously to the dorsal skin of SD rats at a dose of 0.5 mL/head.

In this experiment, the experimental groups G7-1 to G7-3 in Table 10 below, the number of animals included in each experimental group was 9 per group, and 3 animals were assigned to each group on the 1st day, 4th week, and 12th week, respectively.

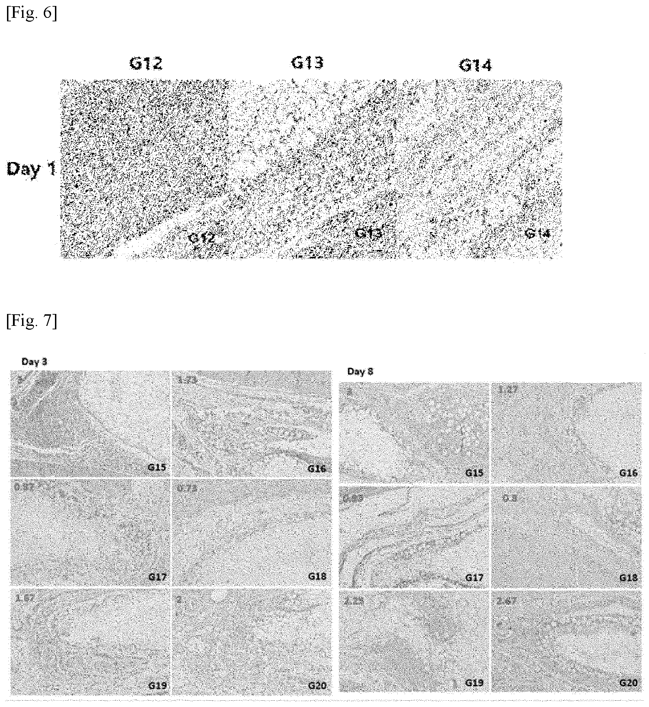

After sacrificing 3 animals in each group on the 1^{st} day, 4^{th} weeks and 12th weeks after administration, the tissues of the administration site were excised to analyze the remaining microspheres, fixed in 10% neutral buffered formalin solution, paraffin-embedded, to make blocks. Four slices were cut to a thickness of about 5 µm and stained with H&E (hematoxylin & eosin) to evaluate the degree of inflammation, such as infiltration of inflammatory cells, angiogenesis, and fibrous tissue formation. The pictures of the stained specimen are shown in FIG. 6.

Fig. 6 is a photograph showing the degree of inflammatory cell infiltration, angiogenesis, and fibrous tissue formation by subcutaneously injecting anti-inflammatory microspheres and leuprolide microspheres into experimental animals, and analyzing inflammation at the microsphere administration site with an optical microscope after H&E (hematoxylin & eosin) staining.

On the first day after administration of leuprolide microspheres and anti-inflammatory microspheres to each experimental group, the degree of inflammatory cell infiltration and the fibrous tissue formation at the site of administration were evaluated in substantially the same manner as in Example 2, to show in Table 10 below. In the table below, the dose of drug microspheres and the dose of anti-inflammatory microspheres are represented as mg/head based on active ingredient content.

**[Table 10]**

| Experimental group | G7-1 | G7-2 | G7-3 |
|---|---|---|---|
| Dose of active ingredient in drug microspheres | 2.7 | 2.7 | 2.7 |
| Dose of active ingredients in anti-inflammatory microspheres | Untreated | 0.06 | 0.27 |
| inflammatory cell infiltration | 3.3 | 2 | 1.3 |
| Angiogenesis | 0 | 0 | 0 |
| formation of fibrous connective tissue | 0 | 0 | 0 |

In Group G7-1 administered with leuprolide microspheres at an amount of 2.7mg/head (based on active ingredient content) alone, an average grade 3.3 of inflammatory cell infiltration was observed on the 1st day after administration when acute inflammation occurred, but angiogenesis and formation of fibrous connective tissue were not observed.

In Group G7-2 administered with 2.7mg/head of leuprolide microspheres (based on active ingredient content) and 0.23mg/head of dexamethasone microspheres (0.06mg/head based on active ingredient content), an average grade 2 of inflammatory cell infiltration was observed on the 1st day after administration when acute inflammation occurred, but angiogenesis and formation of fibrous connective tissue were not observed.

In Group G7-3 administered with 2.7mg/head of leuprolide microspheres (based on the active ingredient content) and 1.04mg/head of dexamethasone microspheres (0.27mg/head based on the active ingredient content), an average grade 1.3 of inflammatory cell infiltration was observed on the 1st day after administration when acute inflammation occurred, but angiogenesis and formation of fibrous connective tissue were not observed.

### 7-2: Combination of placebo microspheres and dexamethasone microspheres

30 µm placebo microspheres prepared in Example 2-1 were used as microspheres for inducing inflammation. In substantially the same manner as in Example 3, microspheres for inducing inflammation alone (G7-4) or a mixed formulation of dexamethasone microspheres (A-1) were prepared (G7-5, G7-6, G7-7).

Each of the prepared experimental formulations was administered subcutaneously to the dorsal side of SD rats at a dose of 120 mg/head. Dexamethasone microspheres (A-1) administered in a mixed formulation with the microspheres for inducing inflammation was 0.033 mg/head (0.012 mg/head based on active ingredient content), 0.164 mg/head (0.06 mg/head based on active ingredient content), or 0.82 mg/head (0.3 mg/head based on active ingredient content).

In order to analyze whether or not the anti-inflammatory effect of dexamethasone was maintained in the administered placebo microspheres, the time points for testing inflammation were day 3 and day 8 after administration in Example 3. At each time point, the histopathological slide specimens of Groups G7-4 to G7-7 were prepared using the same method as in Example 3, and tested for inflammatory cell infiltration, to show the results in FIG. 7 and Table 11. Specifically, the inflammatory cell infiltration at the site of administration was evaluated by H&E staining of histopathological slide specimens. The degree of inflammatory cell infiltration at the administration site was evaluated in substantially the same manner as in Example 3.

### 7-3: Combination of placebo microspheres and dexamethasone drug

In Example 7-2, inflammatory microspheres alone (G7-4) or a mixed formulation of dexamethasone microspheres (A-1) were used. Unlikely, in this test, dexamethasone base or dexamethasone acetate which was not encapsulated in microspheres was mixed with 30 µm placebo microspheres prepared in Example 2-1 as inflammatory microspheres to prepare test formulations (G7-8, G7-9).

In addition, anti-inflammatory drugs of dexamethasone base and dexamethasone acetate which was not encapsulated in microspheres were administered at a dose of 0.06 mg/head based on active ingredient content (G7-8, G7-9). Detailed information on the specific experimental groups (G7-8 and G7-9) was written in Table 11 above. In the following experiment, the number of animals on day 3 in each experimental group was four animals for G7-8 and G7-9.

In order to test whether or not the anti-inflammatory effect of dexamethasone was maintained in the administered placebo microspheres, the time points for inflammation test in Example 3 were day 3^{rd} day and 8^{th} day after administration. At each time point, histopathological slide specimens of G7-8 and G7-9 were prepared and tested for the inflammatory cell infiltration using the same method as in Example 3, to show the results in FIG. 7 and Table 11.

**[Table 11]**

| Experimental group | Dose of inflammatory microspheres | Dose of anti-inflammatory drug (mg/head based on active ingredient content) | Inflammato ry cell infiltration (day 3) | Inflammatory cell infiltration (day 8) |
|---|---|---|---|---|
| G7-4 | 120 mg/head | untreated | 3.00±0.00 | 3.00±0.00 |
| G7-5 | 120 mg/head | A-1 microsphere 0.012mg/head | 1.73±0.43 | 1.27±0.37 |
| G7-6 | 120 mg/head | A-1 microsphere 0.06mg/head | 0.87±0.18 | 0.93±0.28 |
| G7-7 | 120 mg/head | A-1 microsphere 0.3 mg/head | 0.73±0.36 | 0.80±1.05 |
| G7-8 | 120 mg/head | Dexamethasone (free base) 0.06mg/head | 1.67±0.00 | 2.25±0.17 |
| G7-9 | 120 mg/head | Dexamethasone Acetate 0.06mg/head | 2.00±0.27 | 2.67±0.27 |

Numerical values in the table above are shown according to the following criteria.
•Inflammatory cell infiltrate score:

| | |
|---|---|
| 0: | ≤ 5 cells per HPF |
| 1: | 6-20 cells per HPF |
| 2: | 21-50 cells per HPF |
| 3^{:} | > 50 cells per HPF |
| High-Power Field = HPF (Objective lens 40X) | |

(the score is from an average of 5 non-overlapping HPF)

As shown in the analysis results in the table, in Group G7-4 administered with only 120 mg/head of 30 µm placebo microspheres, an average grade 3 of inflammatory cell infiltration was observed on the 3rd day after administration when acute inflammation occurred, and average grade 3 of inflammatory cell infiltration was observed in all animals on the 8th day when chronic inflammation occurred.

In Group 7-5 to Group G7-7 administered with 120 mg/head of 30µm placebo microspheres and 0.03 mg/head of dexamethasone microspheres (0.012 mg/head based on active ingredient content), 0.16mg/head (0.06 mg/head based on active ingredient content), or 0.82 mg/head (0.3mg /head based on active ingredient content), average grade of 0.7 to 1.7 of inflammatory cell infiltration was observed on the 3rd day after administration when acute inflammation occurred, and the average grade 0.8 to 1.3 of Inflammatory cell infiltration was observed on the 8th day when chronic inflammation occurred. As the amount of dexamethasone increased, it was confirmed that inflammatory cell infiltration decreased.

In addition, as an index for the inflammatory response at the site of administration, staining of CD68 as a macrophage marker was performed at the site of administration using an antibody staining. The degree of macrophage infiltration at the administration site is shown in Table 12 below. In Table 12 below, score 1 of distribution indicates 5 to 30% of macrophages which are positive in the microscope, score 2 is for 31 to 60% of macrophages which are positive in the microscope, and score 3 is for 60% or more of macrophages which are positive in the microscope.

**[Table 12]**

| Experimental group | Macrophage distribution (3^{rd} day) | Macrophage distribution (8th day) |
|---|---|---|
| G7-4 | 3 | 2.5 |
| G7-5 | 2 | 1.5 |
| G7-6 | 1 | 1 |
| G7-7 | 1 | 2 |
| G7-8 | 1.5 | 2.5 |
| G7-9 | 1 | 2 |

It was confirmed that the degree of macrophage infiltration on the 3rd day when the acute inflammatory response occurred was reduced in all of the Groups G7-5 to G7-9 co-administered with dexamethasone compared to that of Group G7-4. The macrophage infiltration on 8th day was reduced in all of groups G5 to G9 co-administered with dexamethasone compared to that of Group G7-4. In G7-8 and G7-9 administered with the dexamethasone drug (without drug carrier), the macrophage infiltration pattern increased from the 3rd day.

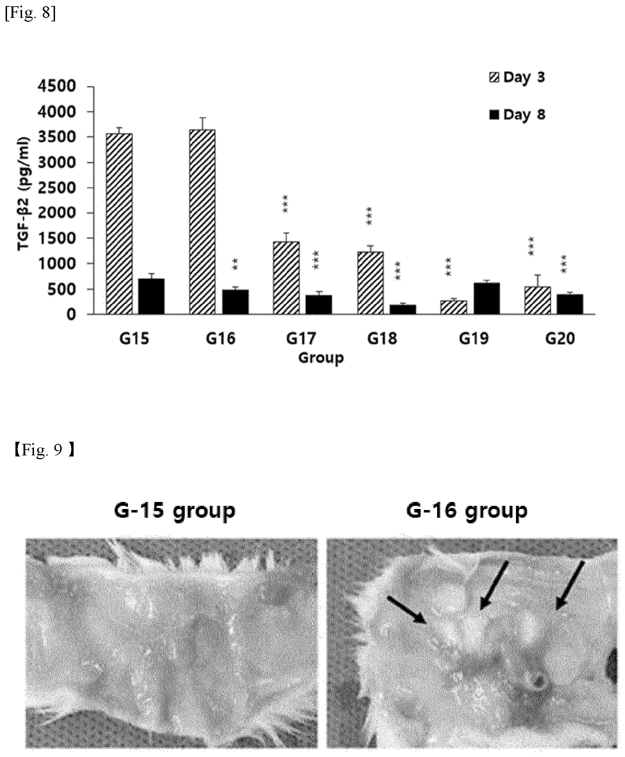

As an index for the systemic inflammatory response, the concentration of TGF-β2 in the blood was analyzed by ELISA method, and the concentration of TGF-β2 in the blood is shown in FIG. 8.

Similar to the histopathology data, it was confirmed that all of the groups G7-5 to G7-9 to which dexamethasone was administered, showed decreased blood concentration of TGF-β2 on the 3rd day when acute inflammation occurred, and showed lower blood concentration of TGF-β2 on the 8th day than that of G7-4 administered with inflammatory microspheres alone. The pattern in which dexamethasone decreases the concentration of TGF-β2 is similar to that in reducing the degree of inflammatory cell infiltration in histopathological data. The anti-inflammatory property of dexamethasone on 8^{th} day was found to be better with dexamethasone in the form of microspheres compared to dexamethasone not encapsulated in microspheres.

### Example 8: Analysis of anti-inflammatory effect for combined formulation

### 8-1: Combined formulation of drug microspheres and anti-inflammatory microspheres

The degree of inflammation in rats was evaluated depending on the administration of drug microspheres alone or combined with anti-inflammatory microspheres. The Used drug microspheres are donepezil microspheres (B-2), rivastigmine microspheres (B-3), finasteride microspheres (B-4), semaglutide microspheres (B-5), leuprolide microspheres (B-8, B- 9), octreotide microspheres (B-7), and deslorelin microspheres (B-11, B-12). Each of deslorelin microspheres and donepezil microspheres was administered in combination with A-2 anti-inflammatory microspheres. Each of rivastigmine microspheres and finasteride microspheres was administered in combination with A-4 anti-inflammatory microspheres. Octreotide microspheres were administered in combination with A-3 anti-inflammatory microspheres. Each of semaglutide microspheres and leuprolide microspheres was administered in combination with A-10 anti-inflammatory microspheres.

Animal experiments were performed according to the Animal Experimentation Ethics Committee regulations. Specifically, after purchasing 6-week-old male SD (Sprague-Dawley) rats and acclimatizing them for 1 week, the test drug was administered to 7-week-old rats. Donepezil microspheres, rivastigmine microspheres, and leuprolide microspheres were administered to the femoral muscle of the right hind limb of rats, and other drug microspheres were administered subcutaneously to the dorsal side of rats. The dose, the volume of administered solution and the animal groups are as follows. In Table 13, the drug dose described in the first part is the drug administration dose of the second formulation, and the drug dose described in the latter part means the administration dose of the anti-inflammatory drug of the first formulation. Also, in drug dose of Table 13 below, the former column refers to the dose of drug contained in the second formulation and the latter column refers to the dose of anti-inflammatory drug contained in the first formulation.

**[Table 13]**

| Group | Second microsphere /First microsphere | Dose of drug in B microsphere (mg/head as API) | Dose of drug in A microsphere (mg/head as API) | Administe red volume (mL/head ) | 3rd day | 8th day |
|---|---|---|---|---|---|---|
| G8-1 | Negative control | - | 0 | - | 2 | - |
| G8-2 | B-11 | 4.7 | 0 | 0.5 | 4 | 4 |
| G8-3 | B-12 | 4.7 | 0 | 0.5 | 4 | 4 |
| G8-4 | B-11 | 4.7 | A-2 (0.06) | 0.5 | 4 | 4 |
| G8-5 | B-2 | 26.04 | 0 | 0.3 | 4 | 4 |
| G8-6 | B-2 | 26.04 | A-2 (0.03) | 0.3 | 4 | 4 |
| G8-7 | B-3 | 8.68 | 0 | 0.21 | 4 | 4 |
| G8-8 | B-3 | 8.68 | A-4 (0.03) | 0.21 | 4 | 4 |
| G8-9 | B-4 | 8.4 | | 0.3 | 4 | 4 |
| G8-10 | B-4 | 8.4 | A-4 (0.018) | 0.3 | 4 | 4 |
| G8-11 | B-5 | 3.6 | 0 | 0.5 | 4 | 4 |
| G8-12 | B-5 | 3.6 | A-10 (0.054) | 0.5 | 4 | 4 |
| G8-13 | B-8 | 1.5 | 0 | 0.3 | 4 | 4 |
| G8-14 | B-9 | 1.5, 0.023 | 0 | 0.3 | 4 | 4 |
| G8-15 | B-8 | 1.5 | A-10 (0.024) | 0.3 | 4 | 4 |
| G8-16 | B-7 | 3 | 0 | 0.5 | 4 | 4 |
| G8-17 | B-7 | 3 | A-3 (0.03) | 0.5 | 4 | 4 |

On the 3rd and 8th day after administration, four rats per group were sacrificed, and the region of administration was excised to analyze the difference in the degree of inflammation caused by administration of drug microspheres alone and in combination with anti-inflammatory microspheres in the acute and chronic inflammatory phases.

In order to evaluate the degree of inflammation, the excised administration region was fixed and embedded in neutral formalin, to make a paraffin block. The paraffin block was cut into 4 to 5 µm thick slices. After staining with H&E (hematoxylin & eosin), inflammatory cells were investigated using an optical microscope. The degree of infiltration, angiogenesis and fibrous tissue formation was analyzed.

The degree of inflammatory cell infiltration, fibrous tissue formation, and angiogenesis are shown in Table 14 below. Using an optical microscope on histopathological slide of the stained tissue, three fields were selected randomly in 400x magnification field of view to investigate the infiltration of inflammatory cells, and the numbers were represented as "grade 0" for≤5(or lower) of number, "grade 1" was represented for 6 to 20, "grade 2"was represented for 21 to 50, and "grade 3" was represented for over 50. In Table 14, they were represented as the average value of each group. In addition, the case that the fibrous tissue formation and angiogenesis were not observed was represented as "grade 0", "grade 1" represented weak case, "grade 2" represented medium case, "grade 3" represented strong case, and "grade 4" represented serious case.

**[Table 14]**

| Experim ental group | Inflammatory cell infiltration at 3^{rd} day | Fibrous tissue formation at 3^{rd} day | Angiogenes is at 3^{rd} day | Inflammator y cell infiltration at 8^{th} day | Fibrous tissue formation at 8^{th} day | Angiogenesis at 8^{th} day |
|---|---|---|---|---|---|---|
| G8-1 | 0.00±0.00 | 0.00±0.00 | 0.00±0.00 | - | - | - |
| G8-2 | 2.08±0.25 | 1.67±0.41 | 1.42±0.14 | 1.50±0.50 | 1.92±0.60 | 2.00±0.41 |
| G8-3 | 0.50±0.00 | 0.58±0.43 | 0.92±0.43 | 0.67±0.41 | 0.75±0.14 | 0.83±0.17 |
| G8-4 | 0.33±0.42 | 0.50±0.17 | 1.25±0.55 | 0.22±0.16 | 0.56±0.16 | 0.33±0.00 |
| G8-5 | 2.17±0.25 | 1.42±0.14 | 1.75±0.28 | 0.75±0.28 | 1.25±0.28 | 0.08±0.14 |
| G8-6 | 1.56±0.00 | 0.44±0.16 | 0.44±0.16 | 0.33±0.00 | 1.50±0.17 | 0.25±0.28 |
| G8-7 | 1.89±0.00 | 1.67±0.27 | 0.67±0.27 | 0.17±0.17 | 0.75±0.14 | 0.08±0.14 |
| G8-8 | 1.42±0.29 | 1.08±0.28 | 0.25±0.14 | 0.00±0.00 | 0.50±0.17 | 0.00±0.00 |
| G8-9 | 2.67±0.25 | 0.50±0.29 | 0.92±0.60 | 1.44±0.57 | 0.56±0.31 | 1.33±0.27 |
| G8-10 | 1.17±0.42 | 0.92±0.89 | 1.00±0.58 | 0.22±0.16 | 0.33±0.00 | 0.89±0.16 |
| G8-11 | 1.67±0.00 | 1.00±0.00 | 0.50±0.17 | 1.33±0.33 | 0.83±0.50 | 1.83±0.37 |
| G8-12 | 0.67±0.29 | 0.33±0.33 | 0.67±0.67 | 0.56±0.31 | 0.33±0.00 | 1.00±0.27 |
| G8-13 | 2.58±0.00 | 1.25±0.64 | 1.08±0.64 | 1.75±0.43 | 2.00±0.24 | 1.83±0.37 |
| G8-14 | 0.56±0.00 | 0.00±0.00 | 0.00±0.00 | 0.08±0.14 | 0.83±0.17 | 0.58±0.28 |
| G8-15 | 0.58±0.25 | 0.42±0.28 | 0.42±0.28 | 0.08±0.14 | 0.17±0.17 | 0.42±0.14 |
| G8-16 | 1.83±0.25 | 2.08±0.14 | 0.42±0.36 | 2.00±0.47 | 2.22±0.16 | 0.56±0.79 |
| G8-17 | 0.00±0.00 | 0.44±0.16 | 0.00±0.00 | 0.00±0.00 | 0.17±0.17 | 0.00±0.00 |

The inflammatory cell infiltration, angiogenesis, and fibrous tissue formation in subcutaneous or muscle tissue were significantly observed in the group administered with the test substance compared to those of G8-1 control group on the 3rd day after drug microsphere administration, and the degree of inflammatory response was different depending on test substances. The inflammatory response tended to decrease in the group administered in combined with anti-inflammatory microspheres. A similar tendency was observed in the investigation on the 8th day of administration. When dexamethasone as an anti-inflammatory drug was encapsulated with the drug in the second drug delivery system, the inflammatory cell infiltration, the angiogenesis and the fibrous tissue formation were reduced similarly to the case where dexamethasone encapsulated in the first drug delivery system was administered together with the second drug delivery system in the combined microspheres.

### 8-2: Combination of drug microspheres and anti-inflammatory drugs

The degree of inflammation in rats for semaglutide microspheres was tested with or without co-administration of meloxicam. Specifically, 8-week-old male SD (Sprague-Dawley) rats were purchased and acclimatized for one week, and then the test substance was administered subcutaneously to dorsal side of 9-week-old rats. The semaglutide microspheres (B-6) were administered at 3.6mg/head and meloxicam at 1.5mg/head in a volume of 0.5mL, respectively.

On the 3rd day after administration, one rat per group was sacrificed, and the injection region was excised to analyze the difference in the degree of inflammation in the acute inflammation stage. The excised tissue was stained by the H&E method of Example 8-1, and the degree of inflammatory cell infiltration, angiogenesis, and fibrous tissue formation was analyzed using the same criteria.

Table 15 below shows the degree of inflammatory cell infiltration, fibrous tissue formation, and angiogenesis. It was confirmed that the meloxicam drug itself effectively reduced inflammation caused by semaglutide microspheres.

**[Table 15]**

| Experimental group | Inflammatory cell infiltration | Fibrous tissue formation | Angiogenesis |
|---|---|---|---|
| B-6 | 2 | 2 | 2 |
| B-6 and meloxicam | 1 | One | 0 |

### Example 9. Evaluation for drug release control

### 9-1: Delayed degradation of biodegradable polymers by dexamethasone

30 µm placebo microspheres alone (G9-1 group) prepared in Example 2-1 or a mixture (G7-5 group) of the placebo microspheres and dexamethasone microspheres (A-1) prepared in Example 1 were administered subcutaneously to the dorsal side of SD rats, and then the degradation degree of placebo microspheres in the groups was compared. On the 16th day after administering the placebo microspheres alone or the mixture of placebo microspheres and dexamethasone microspheres, the tissues were excised from the administration region to analyze residual microspheres. As a result, the degradation of biodegradable microspheres was delayed in the combined administration group of placebo microspheres and dexamethasone microspheres (A-1), compared to the group administered with placebo microspheres alone. A photograph of the rat obtained as a result of the above experiment is shown in FIG. 9.

### 9-2: Delayed degradation of drug microspheres by dexamethasone

In the same manner as in Example 9-1, the microspheres containing leuprolide, or a mixture of leuprolide microspheres and dexamethasone microspheres (A-1) were subcutaneously once to the dorsal side of SD rats. This experiment are referred to experimental groups G9-3 to G9-5 in Table 17 below, and the number of animals included in each experimental group is 3 per group.

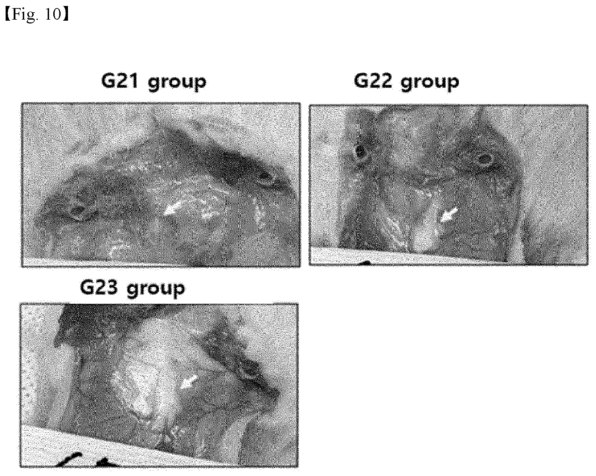

In Table 17, the amount of residual microspheres was analyzed by calculating the area through image J open source software (version 1.45s, NIH). The area in the experimental group G9-3 was represented as 1 (+), and an increase of 1 to 100% based on the amount of residual microspheres in experimental group G9-3 was marked with ++, and an increase of 101% to 200% was marked with +++. A photograph of the rat obtained as a result of the above experiment is shown in FIG. 10. Three animals were used in each experimental group of Table 16 below.

**[Table 16]**

| Experimental group | Dose of active ingredient in drug microspheres (mg/head, API) | Dose of active ingredients in anti-inflammatory microspheres (mg/head, API) | Amount of residual microspheres |
|---|---|---|---|
| G9-3 | 2.7 mg/head | 0 mg/head | + |
| G9-4 | 2.7 mg/head | 0.06 mg/head | ++ |
| G9-5 | 2.7 mg/head | 0.27 mg/head | +++ |

After sacrificing the rats at 4th weeks after administration of the test substance, tissues at the site of administration were excised to analyze the remaining microspheres. As a result, the amount of residual microspheres were large in the groups (G9-4 and G9-5) administered with the dexamethasone microspheres, and it confirmed that the amount of residual microspheres increased as the amount of dexamethasone increased. According to the result, dexamethasone delayed the degradation of drug microspheres, and use of this property could control the drug release properties of drug microspheres.

### Example 10. Bioavailability (AUC) improvement by combined administration of drug microspheres and anti-inflammatory microspheres

For the anti-inflammatory microspheres A-2, A-3, and A-4 prepared in Example 1, using 9-week-old SD (Sprague-Dawley) rats, the donepezil microspheres (B-2), rivastigmine microspheres (B-3) and semaglutide microspheres (B-5) prepared in Example 2, and octreotide microspheres named as B-15 which was 1-month acting microspheres purchased as commercially available Sandostatin Lar, and used in this experiment. In addition, as described in Example 7, a pharmacokinetic evaluation test of combined administrated drugs was performed with leuprolide microspheres (B-14) purchased as a commercial product.

In the case of donepezil microspheres, 26.04 mg/head (API) was mixed with 0.3 mg/head (API) of anti-inflammatory microsphere A-2 and intramuscularly administered.

In the case of rivastigmine microspheres, 8.64 mg/head (API) was mixed with 0.03 mg/head (API) or 0.01 mg/head (API) of anti-inflammatory microspheres A-4 and intramuscularly administered.

In the case of finasteride microspheres (B-4), 8.4 mg/head (API) was mixed with 0.018 mg/head (API) of anti-inflammatory microspheres A-4 and administered subcutaneously.

In the case of semaglutide microspheres (B-5), 3.6 mg/head (API) was mixed with 0.054 mg/head (API) of anti-inflammatory microspheres A-4 and administered subcutaneously.

In the case of semaglutide microspheres (B-5), 3.6 mg/head (API standard) was mixed with 0.054 mg/head (API) of anti-inflammatory microsphere A-10 and administered subcutaneously.

In the case of octreotide microspheres (B-15), 3 mg/head (API) was mixed with 0.03 mg/head (API) of anti-inflammatory microspheres A-4 and administered intramuscularly.

In the case of leuprolide microspheres (B-14), 4.5 mg/head (API) was mixed with 0.072 mg/head (API) of anti-inflammatory microspheres A-3 and administered subcutaneously.

In the case of deslorelin microspheres (B-11), 4.7 mg/head (API) was mixed with 0.06 mg/head (API) of anti-inflammatory microsphere A-2 and administered subcutaneously.

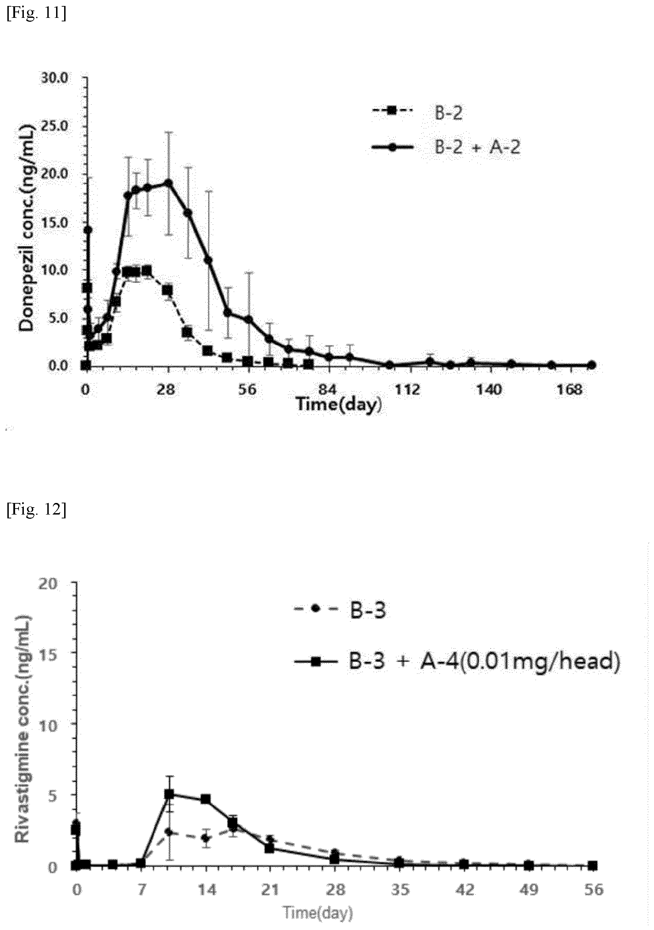

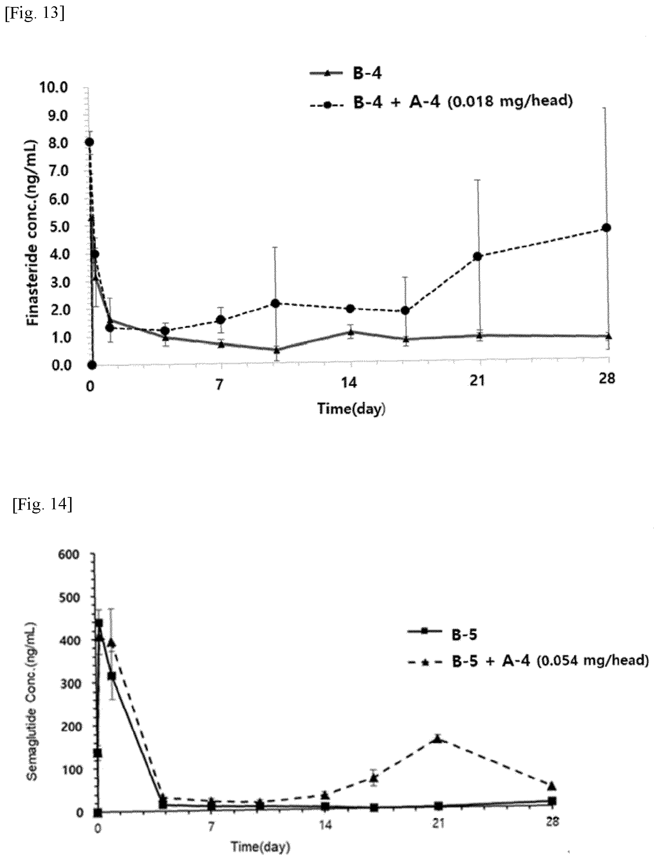

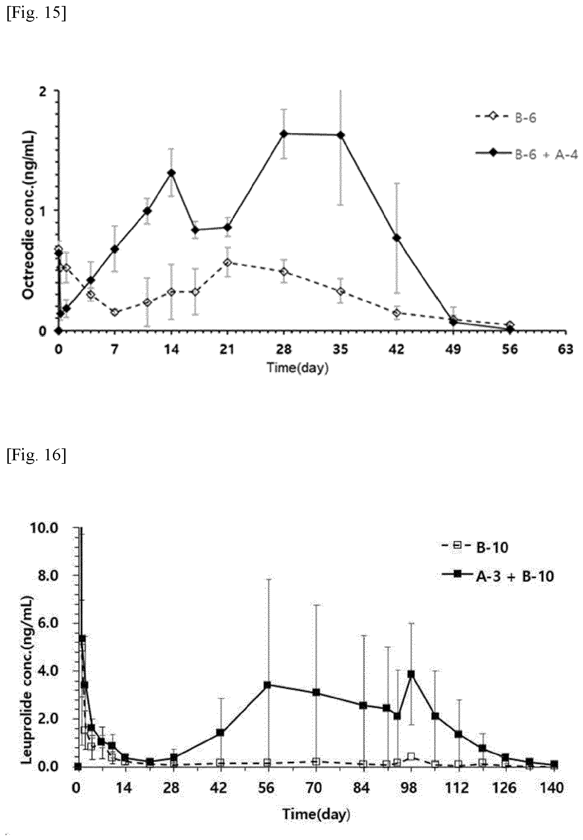

0.25 to 0.5 mL of blood was collected at each predetermined time points, and the concentration of drug microspheres in blood was measured using LC-MS/MS, to the results in graphs of FIGs. 11 to 16.

In Table 17 below, the dose of drug microspheres and the dose of anti-inflammatory microspheres refer to the amount (mg/head) of the active ingredient administered by administration of the microspheres.

**[Table 17]**

| Experimental group | Dose of active ingredient in drug microspheres (mg/head, API) | Type | Dose of active ingredients in anti-inflammatory microspheres (mg/head, API) | Type |
|---|---|---|---|---|
| G10-1 | 26.04 | B-2 | 0 | ** |
| G10-2 | 26.04 | B-2 | 0.3 | A-2 |
| G10-3 | 8.68 | B-3 | 0 | ** |
| G10-4 | 8.68 | B-3 | 0.03 | A-4 |
| G10-5 | 8.4 | B-4 | 0 | ** |
| G10-6 | 8.4 | B-4 | 0.018 | A-4 |
| G10-7 | 3.6 | B-5 | 0 | ** |
| G10-8 | 3.6 | B-5 | 0.054 | A-4 |
| G10-9 | 3 | B-15 | 0 | ** |
| G10-10 | 3 | B-15 | 0.03 | A-4 |
| G10-11 | 4.5 | B-14 | 0 | ** |
| G10-12 | 4.5 | B-14 | 0.072 | A-3 |

In the case of donepezil microspheres, when administered in combination with anti-inflammatory microspheres A-2 (A-2 & B-2), the AUC improvement was 277% compared to the single administration group, and the drug release time was increased. In the case of rivastigmine microspheres, when co-administered with anti-inflammatory microspheres A-4 (A-4 & B-3), the AUC improvement was 26% compared to the group administered alone.

In the case of finasteride microspheres, when combined with anti-inflammatory microspheres A-4 (A-4 & B-4), the AUC improvement was 461% compared to the group administered alone.

In the case of semaglutide microspheres, when combined with anti-inflammatory microspheres A-4 (A-4 & B-5), the AUC improvement was 290% compared to the group administered alone.

In the case of octreotide microspheres, when anti-inflammatory microspheres A-4 were co-administered (A-4 & B-15), the AUC improvement was 287% compared to the group administered alone.

In the case of leuprolide microspheres, when administered in combination with anti-inflammatory microspheres A-3 (A-3 & B-14), that not only the AUC improvement was improved to 293% compared to the group administered alone, but also the drug release period was increased.

In the case of deslorelin microspheres, when administered in combination with anti-inflammatory microspheres A-2 (A-2 & B-11), the AUC improvement was 509% compared to the group administered alone.

As a result of the above, anti-inflammatory microspheres A-2, A-3, and A-4 microspheres were co-administered with drug microspheres, the bioavailability (AUC) of donepezil, rivastigmine, finasteride, leuprolide, semaglutide, octreotide or deslorelin was improved.

### Example 11. Bioavailability (AUC) improvement by combined administration of semaglutide microspheres and anti-inflammatory drugs

Using 9-week-old SD (Sprague-Dawley) rats, the bioavailability improvement property of semaglutide was tested by co-administration of semaglutide microspheres (B-6) prepared in Examples 2-6 in combination with anti-inflammatory microspheres prepared in Example 1 (G3 to G6 and G9 in Table 18 below).

In addition, the bioavailability improvement property of semaglutide was tested by co-administration of mixed formulations of semaglutide microspheres (B-6) prepared in Examples 2-6 and each of dexamethasone base, dexamethasone acetate and meloxicam which were not encapsulated in the microspheres (G7, G8, G12 in the table below)

In substantially the same manner as in Example 9, the mixed formulations were administered to rats, and 0.25 to 0.5 mL of blood was collected at predetermined times for 28 days, and the concentration of semaglutide in blood was analyzed using LC-MS/MS. Based on the measured drug concentration, Cmax and AUC were calculated and shown in the table below. In the table below, the semaglutide microspheres used in G11 and G12 are B-5 microspheres prepared in Example 2, and the semaglutide microspheres used in G3 to G8 are B-6 microspheres prepared in Example 2.

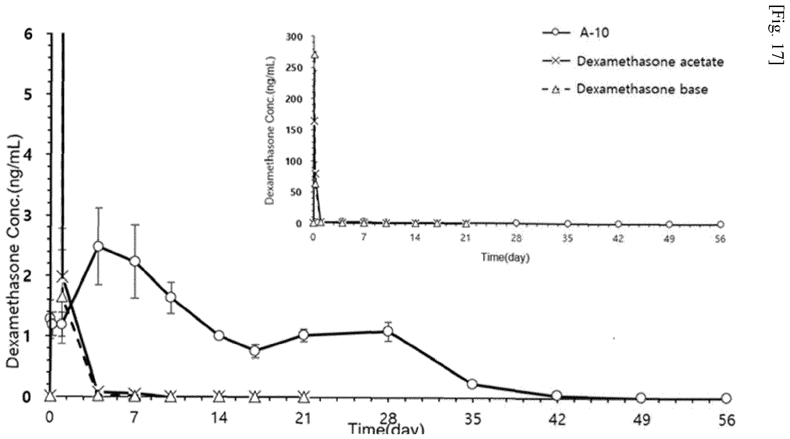

The experimental results are shown in Table 18 and FIG. 17.

**[Table 18]**

| c | Dose of active ingredient in drug microspheres (mg/head, API) | Dose of active ingredients in anti-inflammatory microspheres (mg/head, API) | Types of anti-inflammator y drugs | Cmax (ng/mL) | AUCnorm (ng*day/mL)/( mg/kg) |
|---|---|---|---|---|---|
| G11 | 3.6 | 0 | no | 440.1 | 166.3 |
| G12 | 3.6 | 0.054 | A-5 | 406.9 | 354.1 |
| G3 | 3.6 | 0 | no | 308.6 | 402 |
| G4 | 3.6 | 0.054 | A-10 microspheres | 1015.8 | 1423.7 |
| G5 | 3.6 | 0.054 | A-6 microspheres | 929.4 | 888.2 |
| G6 | 3.6 | 0.108 | A-10 microspheres | 1107.3 | 1606.7 |
| G9 | 3.6 | 0.108 | A-4 microspheres | 1099.2 | 1596.2 |
| G7 | 3.6 | 0.108 | Dexamethas one Acetate | 627.3 | 816.1 |
| G8 | 3.6 | 0.108 | Dexamethas one Base | 829.5 | 791.8 |

As shown in the table above, when semaglutide microspheres were administered alone without anti-inflammatory microspheres or anti-inflammatory drugs (G3), AUC was 402.0 (ng*day/mL)/(mg/kg). However, in the mixed formulation of semaglutide microspheres and anti-inflammatory microspheres containing dexamethasone acetate (G4, G5, G6), their AUC were 1423.7, 888.2, or 1606.7 (ng*day/mL)/(mg/kg), respectively, which showed notably higher value compared to G3 group administered by semaglutide microspheres alone, indicating excellent bioavailability. In addition, even in combined microsphere formulation of the microspheres containing dexamethasone acetate, G6 group for the anti-inflammatory microspheres containing active ingredients at a higher content in the microsphere, has a higher bioavailability than G4 group administered at 0.054 mg/head as dexamethasone acetate. Also, even in combined microsphere formulation of semaglutide microspheres and microspheres containing dexamethasone acetate, when G4 group of 1-month dosage was compared with G5 group of 7-day dosage at the same dose of dexamethasone (0.054 mg/head), G4 group of 1-month dosage showed a higher AUC.

Regarding the mixed formulations (G7, G8) of semaglutide microspheres (B-6) and each of dexamethasone base and dexamethasone acetate which were not encapsulated in the microspheres, even if the dose of the anti-inflammatory drug administered was the same as 0.108 mg/head, G6 group which used combined formulation of semaglutide microspheres (B-6) and microspheres containing dexamethasone acetate (A-10) showed significantly improved AUC. Thus, it confirmed that the combined administration of semaglutide microspheres and anti-inflammatory microspheres was more preferable.

In the graph, the release patterns of dexamethasone in the G6, G7, and G8 groups are shown. The drug release of G7 and G8 terminated in a short period of time, while the drug release of A-10 in the G6 group was maintained at less than 3 ng/head for more than 28 days. The AUC of semaglutide in G6 group was 1606.7 ng*day/mL/mg/kg, which was about twice as high as that of G7 or G8. According to the results, the anti-inflammatory microspheres with a low and long release pattern of dexamethasone are excellent at improving the AUC of semaglutide.

### Example 12. Bioavailability (AUC) improvement by combined administration of deslorelin microspheres and anti-inflammatory drugs

Using 9-week-old SD (Sprague-Dawley) rats, the bioavailability improvement property of deslorelin was tested by co-administration of deslorelin microspheres B-11 prepared in Example 2 in combination with anti-inflammatory microspheres prepared in Example 1.

In substantially the same manner as in Example 9, the mixed formulation was administered to rats, and 0.25 to 0.5 mL of blood was collected at predetermined times for 98 days, and the concentration of deslorelin in blood was measured using LC-MS/MS. Based on the measured drug concentration, Cmax and AUC were calculated and shown in the table below. It was confirmed that the bioavailability improvement of deslorelin co-administered with anti-inflammatory microspheres was remarkable compared to the group administered with deslorelin microspheres alone. In particular, various PK profiles showed depending on the raw material of the anti-inflammatory formulation and the type of microspheres, and all of them showed significantly improved AUC compared to the group administered with deslorelin microspheres alone.

**[Table 19]**

| Experimenta l group | Dose of active ingredient in drug microsphere s (mg/head, API) | Dose of active ingredients in anti-inflammatory microspheres (mg/head, API) | Types of anti-inflammatory formulattion | Cmax (ng/mL) | AUCnorm (ng*day/mL)/( mg/kg) |
|---|---|---|---|---|---|
| G12-1 | 4.7 | 0 | ** | 16.2 | 2.37 |
| G12-2 | 4.7 | 0.06 | A-2 microspheres | 27.3 | 12.08 |
| G12-3 | 4.7 | 0.06 | A-7 microspheres | 20.6 | 17.66 |
| G12-4 | 4.7 | 0.06 | A-9 microspheres | 21.3 | 10.42 |
| G12-5 | 4.7 | 0.06 | A-8 microspheres | 16.8 | 10.05 |

### Example 13. Analysis for toxic concentration of Dexamethasone

Microspheres A-13 to A-16 of dexamethasone microspheres and microspheres A-17 to A-22 of dexamethasone acetate microspheres in Table 20 were prepared in the substantially same method of those of anti-inflammatory microspheres A-2 (Example 1-1) and A-5 (Example 1-2), respectively, except that the disperse phase manufacturing conditions and the amount of continuous phase solution in Table 20 were changed.

**[Table 20]**

| Microspher e | polymer | Drug | Used amou nt of drug (g) | Used amou nt of poly mer (g) | Used amoun t of dispers e phase solvent (g) | Used amoun t of contin uous phase solutio n (mL) | Drug content (w/w%) | Encaps ulation efficie ncy of drug (%) |
|---|---|---|---|---|---|---|---|---|
| A-13 | RG503H | Dexamethasone | 0.88 | 2.62 | 13.13 | 2,626 | 21.1 | 83.9 |
| A-14 | RG653H | Dexamethasone | 0.88 | 2.62 | 13.13 | 2,626 | 20.5 | 81.5 |
| A-15 | RG752H:RG75 3H (=30:70) | Dexamethasone | 0.45 | 2.55 | 12.75 | 2,550 | 12 | 80 |
| A-16 | RG752H:RG75 3H (=30:70) | Dexamethasone | 0.88 | 2.62 | 13.13 | 2,626 | 20.7 | 82.3 |
| A-17 | 7502A:7504A (=33:66) | Dexamethasone acetate | 0.4 | 0.6 | 5.5 | 2,000 | 39.8 | 99.4 |
| A-18 | 7502A:7504A(= 50:50) | Dexamethasone acetate | 0.4 | 0.6 | 5.5 | 2,000 | 37.2 | 93 |
| A-19 | 7502A:7504A(= 66:33) | Dexamethasone acetate | 0.4 | 0.6 | 5.5 | 2,000 | 43 | 107.6 |
| A-20 | 6504A:04A(=50 :50) | Dexamethasone acetate | 0.4 | 0.6 | 5.5 | 2,000 | 40.7 | 101.7 |
| A-21 | 6504A:04A(=66 :33) | Dexamethasone acetate | 0.4 | 0.6 | 5.5 | 2,000 | 43.5 | 108.7 |
| A-22 | PDL04A | Dexamethasone acetate | 0.4 | 0.56 | 5.3 | 2,000 | 40.1 | 100.3 |

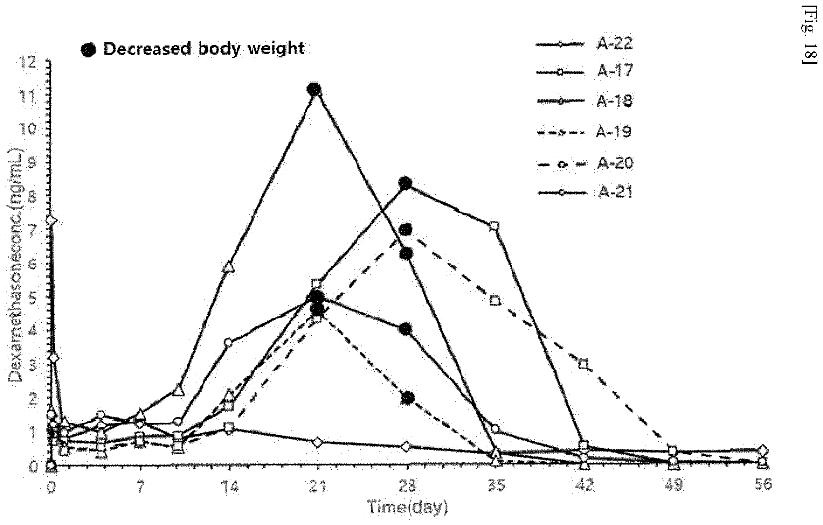

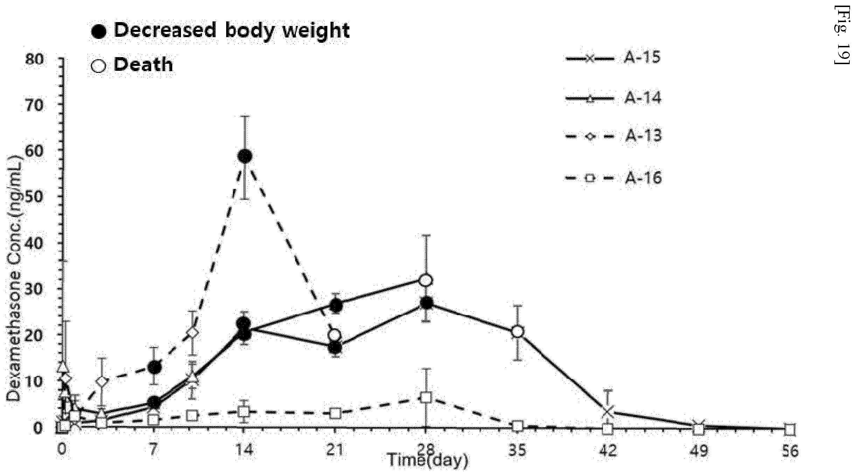

The prepared dexamethasone acetate microspheres or dexamethasone microspheres were administered to 9-week-old SD (Sprague-Dawley) rats, in order to test the toxic concentration of dexamethasone. In Experiment A, dexamethasone acetate microspheres were administered subcutaneously to rats at a dose of 0.3 mg/head as an active ingredient, and in Experiment B, dexamethasone microspheres were administered at a dose of 1 mg/head as an active ingredient. 0.25-0.5 mL of blood was collected at pre-scheduled times, and the concentration of dexamethasone in the blood was measured using LC-MS/MS. In addition, the body weight of the rat was measured every week for 28 days, and the body weight and dexamethasone release pattern were shown in FIGs. 18 and 19, and Tables 21 and 22.

**[Table 21] Change % in animal body weight in Experiment A**

| microsphere | 0 days | 7 days | 14 days | 21st | 28 days |
|---|---|---|---|---|---|
| A-22 | 100 | 106.0 | 116.5 | 124.1 | 130.9 |
| A-17 | 100 | 106.7 | 114.9 | 115.2 | 109.7 |
| A-18 | 100 | 103.0 | 105.5 | 98.9 | 93.6 |
| A-19 | 100 | 106.5 | 113.9 | 115.6 | 114.4 |
| A-20 | 100 | 107.1 | 113.1 | 112.7 | 108.8 |
| A-21 | 100 | 105.9 | 110.7 | 107.4 | 104.6 |

**[Table 22] Change % in animal body weight in Experiment B**

| microsphere | 0 days | 7 days | 14 days | 21st | 28 days |
|---|---|---|---|---|---|
| A-15 | 100 | 101.4 | 91.7 | 80.3 | 73.2 |
| A-14 | 100 | 96.9 | 89.0 | 77.1 | 71.2 |
| A-13 | 100 | 88.7 | 80.4 | 65.0 | Death |
| A-16 | 100 | 104.9 | 111.1 | 111.1 | 111.8 |

Figs. 18 and 19 and the table above, as in A-17, A-18, A-19, A-20, and A-21 of Experiment A, when the blood concentration of dexamethasone was about 4 ng/mL or more for 7 days or more, it was confirmed that the body weight of the rat decreased. In particular, when the blood concentration of dexamethasone was maintained at 10 ng/mL or more for 7 days or more in the high-concentration test of Experiment B, it was confirmed that the body weight of rats decreased, and when the dexamethasone concentration was maintained at 20 ng/mL or more for 7 days or more, the body weight of the rats was significantly reduced or the rats died. In the A-16 microspheres of Experiment B, if the blood concentration of dexamethasone was maintained at 3 ng/mL, it did not cause a decrease in body weight, but normal weight gain did not occur. As in the A-22 microsphere of Experiment A, when the blood concentration of dexamethasone was maintained below 2 ng/mL, the body weight of the rats did not decrease and seemed to show normal weight gain.

## Claims

1. A pharmaceutical kit for combined parenteral administration, comprising a first formulation containing a first drug, and a second formulation containing a second drug and a parenteral drug delivery system,
wherein the first formulation contains a first anti-inflammatory drug, or a first anti-inflammatory drug loaded in a first parenteral drug delivery system,
wherein the second formulation contains a second parenteral drug delivery system and a second drug being different from the first drug, and
wherein the first formulation is for preventing, reducing or treating an inflammatory reaction of a subject caused by the parenteral drug delivery system of the second formulation.

2. A pharmaceutical kit for combined parenteral administration comprising a first formulation containing a first drug, and a second formulation containing a second drug and a parenteral drug delivery system,
wherein the first formulation contains a first anti-inflammatory drug, or a first anti-inflammatory drug loaded in a first parenteral drug delivery system,
wherein the second formulation contains a second parenteral drug delivery system and a second drug different from the first drug, and
wherein the first formulation is for controlling decomposition of the parenteral drug delivery system contained in the second formulation.

3. A pharmaceutical kit for combined parenteral administration comprising a first formulation containing a first drug, and a second formulation containing a second drug and a parenteral drug delivery system,
wherein the first formulation contains a first anti-inflammatory drug, or a first anti-inflammatory drug loaded in a first parenteral drug delivery system,
wherein the second formulation contains a second parenteral drug delivery system and a second drug different from the first drug, and
wherein the first formulation is for increasing bioavailability of the second drug contained in the second formulation.

4. The pharmaceutical kit according to any one of claims 1 to 3, wherein the second parenteral drug delivery system is selected from the group consisting of biodegradable polymers and biodegradable lipids, and induces inflammation in the subject.

5. The pharmaceutical kit according to any one of claims 1 to 3, wherein the drug delivery system is microspheres, liposomes, micelles, depots, or hydrogels.

6. The pharmaceutical kit according to any one of claims 1 to 3, wherein the first formulation and the second formulation are administered at the same time or at different times.

7. The pharmaceutical kit according to any one of claims 1 to 3, wherein the first formulation and the second formulation are provided as a mixed formulation or a separate formulation.

8. The pharmaceutical kit according to any one of claims 1 to 3, wherein the first drug and the second drug are loaded together in the second parenteral drug delivery system.

9. The pharmaceutical kit according to any one of claims 1 to 3, comprising a mixed formulation of the first drug; and the second formulation containing the second parenteral drug delivery system and the second drug.

10. The pharmaceutical kit according to any one of claims 1 to 3, wherein the first drug and the second drug are separately loaded in parenteral drug delivery systems made of the same material or different materials.

11. The pharmaceutical kit according to any one of claims 1 to 3, comprising the first formulation containing the first drug loaded in the first parenteral drug delivery system, and the formulation containing the second drug loaded in the second parenteral drug delivery system.

12. The pharmaceutical kit according to any one of claims 1 to 3, when the first formulation and the second formulation are provided as a mixed formulation, the anti-inflammatory drug is contained in an amount of 0.001 to 5.0 w/w %, based on 100% by weight of the solid content of the mixed formulation.

13. The pharmaceutical kit of any one of claims 1 to 3, when the first formulation and the second formulation are provided as a mixed formulation, and the first formulation contains the first anti-inflammatory drug loaded in the first parenteral drug delivery system, the first parenteral drug delivery system is contained in an amount of 0.002 to 20 w/w%, based on 100% by weight of the solid content of the mixed formulation,

14. The pharmaceutical kit according to any one of claims 1 to 3, wherein the solid content of the first formulation is 0.002 to 20 parts by weight, based on 100 parts by weight of the solid content of the second formulation.

15. The pharmaceutical kit according to claim 1, wherein AUC at the concentration higher than the lowest effective concentration of the anti-inflammatory drug, is 10% or less of AUC of daily dosage form.

16. The pharmaceutical kit of claim 1, wherein the parenteral drug delivery system is provided for intra-articular, subcutaneous, intradermal, intramuscular, intratumoral, intraocular, intravitreal or intratympanic administration.

17. The pharmaceutical kit according to any one of claims 1 to 3, wherein the drug delivery system is microspheres having an average particle diameter of 10 to 100 micrometers.

18. The parenteral anti-inflammatory kit according to any one of claims 1 to 13, further comprising a local anesthetic component.
